(19) **Europäisches Patentamt / European Patent Office / Office européen des brevets**

(11) **EP 4 674 867 A2**

(12) ## EUROPEAN PATENT APPLICATION

(43) Date of publication:
**07.01.2026 Bulletin 2026/02**

(21) Application number: **25187324.6**

(22) Date of filing: **03.07.2025**

(51) International Patent Classification (IPC):
**C07K 16/18** $^{(2006.01)}$

(52) Cooperative Patent Classification (CPC):
**C07K 16/18;** C07K 2317/33; C07K 2317/34;
C07K 2317/92

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **03.07.2024 CN 202410890870**

(71) Applicants:
- **Shenzhen Mindray Bio-Medical Electronics Co., Ltd.**
  **Shenzhen, Guangdong 518057 (CN)**
- **Hytest Ltd.**
  **20520 Turku (FI)**

(72) Inventors:
- **POSTNIKOV, Alexander B.**
  **117186 Moscow (RU)**
- **MOLCHANOVA, Tatiana A.**
  **117186 Moscow (RU)**
- **BEREZNIKOVA, Anastasia V.**
  **117186 Moscow (RU)**
- **RUSANOVA, Anna V.**
  **117186 Moscow (RU)**
- **DERGOUSOVA, Elena A.**
  **117186 Moscow (RU)**
- **KOZLOVSKY, Stanislav V.**
  **117186 Moscow (RU)**
- **GRUZDEVA, Natalia A.**
  **117186 Moscow (RU)**
- **TRUFANOVA, Anna A.**
  **117186 Moscow (RU)**
- **MISHIN, Alexander S.**
  **117186 Moscow (RU)**
- **ROZOV, Fedor N.**
  **117186 Moscow (RU)**
- **KOTLOV, Sergei A.**
  **117186 Moscow (RU)**
- **KATRUKHA, Alexey G.**
  **117186 Moscow (RU)**
- **ZHU, Shibo**
  **Shenzhen 518057 (CN)**
- **ZHAN, Chengxiong**
  **Shenzhen 518057 (CN)**
- **TANG, Tao**
  **Shenzhen 518057 (CN)**
- **WU, Lingjia**
  **Shenzhen 518057 (CN)**

(74) Representative: **KIPA AB**
**Drottninggatan 11**
**252 21 Helsingborg (SE)**

Remarks:
The complete document including Reference Table(s) and the Sequence Listing(s) can be downloaded from the EPO website

(54) ## ANTIBODY OR ANTIGEN-BINDING FRAGMENT THEREOF BINDING TO SS-CTX AND KIT THEREOF

(57) The present application relates to an antibody or an antigen-binding fragment thereof binding to β-CTx. The antibody or an antigen-binding fragment thereof comprises a heavy chain variable region and a light chain variable region. The heavy chain variable region and the light chain variable region comprise HCDR and LCDR sequences of the present application. The present application also relates to a corresponding isolated nucleic acid molecule, expression vector and host cell. In addition, the application also relates to a kit and a method for diagnosing osteoporosis in a subject.

FIG. 1

EP 4 674 867 A2

## Description

### CROSS REFERENCE

This application claims benefit of priority of Chinese Patent Application No. 202410890870.4 filed on July 3, 2024, the content of which is incorporated herein by reference in its entirety.

### TECHNICAL FIELD

The present application relates to the field of diagnosis of osteoporosis and, in particular, to an antibody or antigen-binding fragment thereof binding to β-CTx.

### BACKGROUND

Bone tissue is constantly remodeled through the process of bone resorption and new bone replacement. Osteoclasts are responsible for bone resorption, while osteoblasts are responsible for new bone formation.

In young people, the processes of bone resorption and bone formation remain balanced, but during menopause this balance is disturbed by increased bone loss due to increased absorption. By binding to estrogen receptors on osteoblasts, estrogen directly stimulates the proliferation of osteoblasts and inhibits the differentiation of osteoclasts. In addition, estrogen can also increase regulatory factors produced by osteoblasts, thereby preventing the activity of osteoclasts and further inhibiting bone resorption during menopause (Chubb et al., 2012, Clinical Biochemistry, 45:12, 928-935).

Insufficient calcium intake and low 25-hydroxyvitamin D (25(OH)D) levels may also accelerate age-related bone loss. The reduction in the production of the main precursor of vitamin D in the skin and the synthesis of 1,25(OH)2D (the active metabolite of vitamin D) results in reduced absorption of calcium by the kidneys and this leads to increased secretion of parathyroid hormone (PTH), thus in turn increasing bone absorption.

In the process of synthesizing the main matrix protein Type I collagen, polypeptide chains α1 and α2 are first formed. α1 and α2 combine together in a ratio of 2:1 to form a procollagen molecule having a triple helix structure. Procollagen is secreted into the extracellular environment, in which terminal peptides are isolated from the procollagen, and the resulting immature collagen is incorporated into the fibrous structure. A series of modifications of collagen molecules in the fiber components and their cross-linking with pyridinoline yield mature collagen.

The main degradation product of the Type I collagen C-terminal peptide is a structure composed of two octapeptides (8 amino acid residues, EKAH(β-D)GGR) cross-linked by pyridinoline. In newly formed bones, the octapeptide sequence contains α-aspartic acid, but as the bone ages, this amino acid isomerizes into a β-form (that is, the degree of isomerization is in direct proportion to the age of the bone). Degradation products of Type I collagen, such as the cross-linked Type I collagen carboxy-terminal telopeptide (β-CTx), have been shown to be reliable biomarkers of bone resorption and osteoporosis (Cloos et al., Biochemistry Journal, 2000, 345, 473-480).

Although methods have been developed to measure β-CTx in human serum, due to the short length of the peptide in β-CTx, this has brought great challenges to the screening of anti-β-CTx antibodies, which in turn leads to the limited number of antibodies that specifically bind to β-CTx and further limits the development of β-CTx immunoassays.

In view of this, it is desired to provide a novel antibody specifically binding to β-CTx.

### SUMMARY

In a first aspect, the present application provides an antibody or an antigen-binding fragment thereof binding to β-CTx, comprising:

a heavy chain variable region (VH) and a light chain variable region (VL),
wherein the heavy chain variable region comprises HCDR1 including an amino acid sequence as set forth in any one of SEQ ID NO: 1, 7, 13, 19, 25, 31, 37, 43, 49 or 55 or a variant thereof; HCDR2 including an amino acid sequence as set forth in any one of SEQ ID NO: 2, 8, 14, 20, 26, 32, 38, 44, 50 or 56 or a variant thereof; and HCDR3 including an amino acid sequence as set forth in any one of SEQ ID NO: 3, 9, 15, 21, 27, 33, 39, 45, 51 or 57 or a variant thereof;
wherein, the light chain variable region comprises LCDR1 including an amino acid sequence as set forth in any one of SEQ ID NO: 4, 10, 16, 22, 28, 34, 40, 46, 52 or 58 or a variant thereof; LCDR2 or including an amino acid sequence as set forth in any one of SEQ ID NO: 5, 11, 17, 23, 29, 35, 41, 47, 53 or 59 or a variant thereof; and LCDR3 including an amino acid sequence as set forth in any one of SEQ ID NO: 6, 12, 18, 24, 30, 36, 42, 48, 54 or 60 or a variant thereof; and
wherein each variant comprises one or more amino acid mutations compared to an amino acid sequence from which it is derived, and each amino acid mutation is selected from amino acid substitution, deletion or addition; preferably, the substitution is a conservative substitution; preferably, each variant has at least 80%, at least 90%, or at least 95% identity to the amino acid sequence from which it is derived.

By providing antibodies with specific HCDRs and LCDRs, the present application provides antibodies that specifically bind to β-CTx with excellent binding ability, thereby solving the problem of scarce antibody options for immunoassays of β-CTx to some extent.

In some embodiments, the antibody of the present application comprises:

HCDR1, HCDR2, HCDR3, LCDR1, LCDR2 and LCDR3 including sequences that have at least 80%, at least 90%, or at least 95% identity to SEQ ID NOs: 1-6, respectively;
HCDR1, HCDR2, HCDR3, LCDR1, LCDR2 and LCDR3 including sequences that have at least 80%, at least 90%, or at least 95% identity to SEQ ID NOs: 7-12, respectively;
HCDR1, HCDR2, HCDR3, LCDR1, LCDR2 and LCDR3 including sequences that have at least 80%, at least 90%, or at least 95% identity to SEQ ID NOs: 13-18, respectively;
HCDR1, HCDR2, HCDR3, LCDR1, LCDR2 and LCDR3 including sequences that have at least 80%, at least 90%, or at least 95% identity to SEQ ID NOs: 19-24, respectively;
HCDR1, HCDR2, HCDR3, LCDR1, LCDR2 and LCDR3 including sequences that have at least 80%, at least 90%, or at least 95% identity to SEQ ID NOs: 25-30, respectively;
HCDR1, HCDR2, HCDR3, LCDR1, LCDR2 and LCDR3 including sequences that have at least 80%, at least 90%, or at least 95% identity to SEQ ID NOs: 31-36, respectively;
HCDR1, HCDR2, HCDR3, LCDR1, LCDR2 and LCDR3 including sequences that have at least 80%, at least 90%, or at least 95% identity to SEQ ID NOs: 37-42, respectively;
HCDR1, HCDR2, HCDR3, LCDR1, LCDR2 and LCDR3 including sequences that have at least 80%, at least 90%, or at least 95% identity to SEQ ID NOs: 43-48, respectively;
HCDR1, HCDR2, HCDR3, LCDR1, LCDR2 and LCDR3 including sequences that have at least 80%, at least 90%, or at least 95% identity to SEQ ID NOs: 49-54, respectively; or
HCDR1, HCDR2, HCDR3, LCDR1, LCDR2 and LCDR3 including sequences that have at least 80%, at least 90%, or at least 95% identity to SEQ ID NOs: 55-60, respectively.

In some embodiments, the antibody of the present application comprises:

HCDR1, HCDR2, HCDR3, LCDR1, LCDR2 and LCDR3 including sequences as set forth in SEQ ID NOs: 1-6, respectively;
HCDR1, HCDR2, HCDR3, LCDR1, LCDR2 and LCDR3 including sequences as set forth in SEQ ID NOs: 7-12, respectively;
HCDR1, HCDR2, HCDR3, LCDR1, LCDR2 and LCDR3 including sequences as set forth in SEQ ID NOs: 13-18, respectively;
HCDR1, HCDR2, HCDR3, LCDR1, LCDR2 and LCDR3 including sequences as set forth in SEQ ID NOs: 19-24, respectively;
HCDR1, HCDR2, HCDR3, LCDR1, LCDR2 and LCDR3 including sequences as set forth in SEQ ID NOs: 25-30, respectively;
HCDR1, HCDR2, HCDR3, LCDR1, LCDR2 and LCDR3 including sequences as set forth in SEQ ID NOs: 31-36, respectively;
HCDR1, HCDR2, HCDR3, LCDR1, LCDR2 and LCDR3 including sequences as set forth in SEQ ID NOs: 37-42, respectively;
HCDR1, HCDR2, HCDR3, LCDR1, LCDR2 and LCDR3 including sequences as set forth in SEQ ID NOs: 43-48, respectively;
HCDR1, HCDR2, HCDR3, LCDR1, LCDR2 and LCDR3 including sequences as set forth in SEQ ID NOs: 49-54, respectively; or
HCDR1, HCDR2, HCDR3, LCDR1, LCDR2 and LCDR3 including sequences as set forth in SEQ ID NOs: 55-60, respectively.

In a specific embodiment, an antigen-binding epitope of the antibody or the antigen-binding fragment thereof of the present application is selected from one or more of the group consisting of: AH(betaD)G, AH(betaD)GG, AH(betaD)GGR and AH(betaD)GGR-COOH.

In a second aspect, the present application provides an antibody or an antigen-binding fragment thereof. The antibody can be produced by hybridoma H-215 deposited with VKPM, or can be produced by hybridoma H-216 deposited with VKPM, or can be produced by hybridoma H-217 deposited with VKPM.

In a third aspect, the present application provides an antibody or an antigen-binding fragment thereof. The antibody:

is produced based on a plasmid deposited with VKPM under a deposit number of B-14772 and a plasmid deposited with VKPM under a deposit number of B-14773;
is produced based on a plasmid deposited with VKPM under a deposit number of B-14774 and a plasmid deposited with VKPM under a deposit number of B-14775;
is produced based on a plasmid deposited with VKPM under a deposit number of B-14776 and a plasmid deposited with VKPM under a deposit number of B-14777;
is produced based on a plasmid deposited with VKPM under a deposit number of B-14778 and a plasmid deposited with VKPM under a deposit number of B-14779;
is produced based on a plasmid deposited with VKPM under a deposit number of B-14780 and a plasmid deposited with VKPM under a deposit number of B-14781;
is produced based on a plasmid deposited with VKPM under a deposit number of B-14782 and a plasmid deposited with VKPM under a deposit number of B-14783; or
is produced based on a plasmid deposited with VKPM under a deposit number of B-14784 and a plasmid deposited with VKPM under a deposit number of B-14785.

In a fourth aspect, the present application provides an isolated nucleic acid molecule encoding the antibody or the antigen-binding fragment thereof of the present application.

In a fifth aspect, the present application provides an expression vector comprising the nucleic acid molecule of the present application.

In a sixth aspect, the present application provides a host cell comprising the nucleic acid molecule of the present application or the expression vector of the present application.

In a seventh aspect, the present application provides a kit for detecting β-CTx in a sample, the kit comprising a first antibody or an antigen-binding fragment thereof and a second antibody or an antigen-binding fragment thereof, wherein the first antibody or the second antibody comprises:

a heavy chain variable region (VH) and a light chain variable region (VL),
wherein the heavy chain variable region comprises HCDR1 including an amino acid sequence as set forth in SEQ ID NO: 1, 7, 13, 19, 25, 31, 37, 43, 49 or 55 or a variant thereof; HCDR2 including an amino acid sequence as set forth in SEQ ID NO: 2, 8, 14, 20, 26, 32, 38, 44, 50 or 56 or a variant thereof; and HCDR3 including an amino acid sequence as set forth in SEQ ID NO: 3, 9, 15, 21, 27, 33, 39, 45, 51 or 57 or a variant thereof;
wherein the light chain variable region comprises LCDR1 including an amino acid sequence as set forth in SEQ ID NO: 4, 10, 16, 22, 28, 34, 40, 46, 52 or 58 or a variant thereof; LCDR2 including an amino acid sequence as set forth in SEQ ID NO: 5, 11, 17, 23, 29, 35, 41, 47, 53 or 59 or a variant thereof; and LCDR3 including an amino acid sequence as set forth in SEQ ID NO: 6, 12, 18, 24, 30, 36, 42, 48, 54 or 60 or a variant thereof; and
wherein each variant comprises one or more amino acid mutations compared to an amino acid sequence from which it is derived, and each amino acid mutation is selected from amino acid substitution, deletion or addition; preferably, the substitution is a conservative substitution; preferably, each variant has at least 80%, at least 90%, or at least 95% identity to the amino acid sequence from which it is derived.

In a specific embodiment, the sample is serum or plasma, such as heparin plasma or EDTA plasma, preferably serum or plasma collected after 8 h, 12 h, 24 h, 2 days, 4 days, or 8 days.

In a specific embodiment, the first antibody is different from the second antibody, and each of the first antibody and the second antibody comprises:

HCDR1, HCDR2, HCDR3, LCDR1, LCDR2 and LCDR3 including sequences that have at least 80%, at least 90%, at least 95%, or 100% identity to SEQ ID NOs: 1-6, respectively;
HCDR1, HCDR2, HCDR3, LCDR1, LCDR2 and LCDR3 including sequences that have at least 80%, at least 90%, at least 95%, or 100% identity to SEQ ID NOs: 7-12, respectively;
HCDR1, HCDR2, HCDR3, LCDR1, LCDR2 and LCDR3 including sequences that have at least 80%, at least 90%, at least 95%, or 100% identity to SEQ ID NOs: 13-18, respectively;
HCDR1, HCDR2, HCDR3, LCDR1, LCDR2 and LCDR3 including sequences that have at least 80%, at least 90%, at least 95%, or 100% identity to SEQ ID NOs: 19-24, respectively;
HCDR1, HCDR2, HCDR3, LCDR1, LCDR2 and LCDR3 including sequences that have at least 80%, at least 90%, at least 95%, or 100% identity to SEQ ID NOs: 25-30, respectively;

HCDR1, HCDR2, HCDR3, LCDR1, LCDR2 and LCDR3 including sequences that have at least 80%, at least 90%, at least 95%, or 100% identity to SEQ ID NOs: 31-36, respectively;

HCDR1, HCDR2, HCDR3, LCDR1, LCDR2 and LCDR3 including sequences that have at least 80%, at least 90%, at least 95%, or 100% identity to SEQ ID NOs: 37-42, respectively;

HCDR1, HCDR2, HCDR3, LCDR1, LCDR2 and LCDR3 including sequences that have at least 80%, at least 90%, at least 95%, or 100% identity to SEQ ID NOs: 43-48, respectively;

HCDR1, HCDR2, HCDR3, LCDR1, LCDR2 and LCDR3 including sequences that have at least 80%, at least 90%, at least 95%, or 100% identity to SEQ ID NOs: 49-54, respectively; or

HCDR1, HCDR2, HCDR3, LCDR1, LCDR2 and LCDR3 including sequences that have at least 80%, at least 90%, at least 95%, or 100% identity to SEQ ID NOs: 55-60, respectively.

In some embodiments, the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 1-6, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 25-30, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 37-42, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 25-30, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 25-30, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 31-36, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 31-36, respectively; and the second antibody comprises HCDR1, HCDR2, HCDR3, LCDR1, LCDR2 and LCDR3 including sequences as set forth in SEQ ID NOs: 25-30, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 49-54, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 19-24, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 25-30, respectively; or

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 25-30, respectively.

By selecting a specific combination of the first antibody and the second antibody, the present application provides a kit for detecting β-CTx. The kit has a low cross-reactivity with α-CTx and has a low limit of detection (LOD).

In some embodiments, the percent cross-reactivity of the kit with α-Ctx is not higher than 0.20%, such as not higher than 0.10%, 0.05%, or 0.02%.

In a specific embodiment,

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 1-6, respectively; or

the first antibody comprises HCDR1, HCDR2, HCDR3, LCDR1, LCDR2 and LCDR3 including sequences as set forth in SEQ ID NOs: 1-6, respectively; and the second antibody comprises HCDR1, HCDR2, HCDR3, LCDR1, LCDR2 and LCDR3 including sequences as set forth in SEQ ID NOs: 7-12, respectively.

In some embodiments,

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 37-42, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 31-36, respectively;

the first antibody comprises HCDR1, HCDR2, HCDR3, LCDR1, LCDR2 and LCDR3 including sequences as set forth in SEQ ID NOs: 1-6, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 1-6, respectively; or

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 25-30, respectively.

In a specific embodiment, the first antibody is a capture antibody.

In a specific embodiment, the second antibody is a detection antibody.

In an eighth aspect, the present application provides a method for diagnosing osteoporosis in a subject, including:

using a first antibody or an antigen-binding fragment thereof and a second antibody or an antigen-binding fragment thereof to detect a sample from the subject;

determining the amount of β-CTx in the sample; and

determining whether the subject has osteoporosis based on the determined amount of β-CTx,

wherein one or two of the first antibody and the second antibody comprises:

a heavy chain variable region (VH) and a light chain variable region (VL),

wherein the heavy chain variable region comprises HCDR1 including an amino acid sequence as set forth in SEQ ID NO: 1, 7, 13, 19, 25, 31, 37, 43, 49 or 55 or a variant thereof; HCDR2 including an amino acid sequence as set forth in SEQ ID NO: 2, 8, 14, 20, 26, 32, 38, 44, 50 or 56 or a variant thereof; and HCDR3 including an amino acid sequence as set forth in SEQ ID NO: 3, 9, 15, 21, 27, 33, 39, 45, 51 or 57 or a variant thereof;

wherein the light chain variable region comprises LCDR1 including an amino acid sequence as set forth in SEQ ID NO: 4, 10, 16, 22, 28, 34, 40, 46, 52 or 58 or a variant thereof; LCDR2 including an amino acid sequence as set forth in SEQ ID NO: 5, 11, 17, 23, 29, 35, 41, 47, 53 or 59 or a variant thereof; and LCDR3 including an amino acid sequence as set forth in SEQ ID NO: 6, 12, 18, 24, 30, 36, 42, 48, 54 or 60 or a variant thereof; and

wherein each variant comprises one or more amino acid mutations compared to an amino acid sequence from which it is derived, and each amino acid mutation is selected from amino acid substitution, deletion or addition; preferably, each substitution is a conservative substitution; preferably, the variant has at least 80%, at least 90%, or at least 95% identity to the amino acid sequence from which it is derived.

In a specific embodiment, the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 1-6, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 25-30, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 37-42, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 1-6, respectively;
the first antibody comprises HCDR1, HCDR2, HCDR3, LCDR1, LCDR2 and LCDR3 including sequences as set forth in SEQ ID NOs: 25-30, respectively; and the second antibody comprises HCDR1, HCDR2, HCDR3, LCDR1, LCDR2 and LCDR3 including sequences as set forth in SEQ ID NOs: 7-12, respectively;
the first antibody comprises HCDR1, HCDR2, HCDR3, LCDR1, LCDR2 and LCDR3 including sequences as set forth in SEQ ID NOs: 25-30, respectively; and the second antibody comprises HCDR1, HCDR2, HCDR3, LCDR1, LCDR2 and LCDR3 including sequences as set forth in SEQ ID NOs: 1-6, respectively;
the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 7-12, respectively;
the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 1-6, respectively;
the first antibody comprises HCDR1, HCDR2, HCDR3, LCDR1, LCDR2 and LCDR3 including sequences as set forth in SEQ ID NOs: 31-36, respectively; and the second antibody comprises HCDR1, HCDR2, HCDR3, LCDR1, LCDR2 and LCDR3 including sequences as set forth in SEQ ID NOs: 13-18, respectively;
the first antibody comprises HCDR1, HCDR2, HCDR3, LCDR1, LCDR2 and LCDR3 including sequences as set forth in SEQ ID NOs: 31-36, respectively; and the second antibody comprises HCDR1, HCDR2, HCDR3, LCDR1, LCDR2 and LCDR3 including sequences as set forth in SEQ ID NOs: 25-30, respectively;
the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 13-18, respectively;
the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 49-54, respectively;
the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 19-24, respectively;
the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 25-30, respectively; or
the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 25-30, respectively.

**BRIEF DESCRIPTION OF THE DRAWINGS**

FIG. 1 shows the detection consistency of Kit A (graph at top: heparin lithium VS serum; graph at bottom: EDTA-K2 VS serum).
FIG. 2 shows the detection consistency of Kit B (graph at top: heparin lithium VS serum; graph at bottom: EDTA-K2 VS serum).
FIG. 3 shows the detection consistency of Kit C (graph at top: heparin lithium VS serum; graph at bottom: EDTA-K2 VS serum).
FIG. 4 shows the detection consistency of Kit D (graph at top: heparin lithium VS serum; graph at bottom: EDTA-K2 VS serum).
FIG. 5 shows the detection consistency of Kit E (graph at top: heparin lithium VS serum; graph at bottom: EDTA-K2 VS serum).
FIG. 6 shows the detection consistency of Kit F (graph at top: heparin lithium VS serum; graph at bottom: EDTA-K2 VS serum).
FIG. 7 shows the fitting results of the measured and theoretical values of Kit A.
FIG. 8 shows the fitting results of the measured and theoretical values of Kit B.
FIG. 9 shows the fitting results of the measured and theoretical values of Kit C.

**DETAILED DESCRIPTION**

The technical solutions in the embodiments of the present application are clearly and fully described below.

Obviously, the described embodiments are only merely some, but not all of the embodiments of the present application. Based on the embodiments of the present application, all other embodiments obtained by those of ordinary skill in the art without creative work should fall within the scope of the present application.

Throughout the specification, unless otherwise specified, terms used herein are to be understood as meanings as commonly used in the art. Therefore, unless otherwise defined, all technical and scientific terms used herein have the same meaning as those skilled in the art to which the present application belongs. If a conflict occurs, the specification will take precedence.

As used herein, the term "antibody" refers to an immunoglobulin molecule, including but not limited to monoclonal antibodies, polyclonal antibodies, bispecific antibodies, chimeric antibodies, humanized antibodies and single-chain antibodies. In addition, it also relates to antibodies that are recombinantly or synthetically produced/synthesized.

As used herein, the term "monoclonal antibody" refers to a highly homogeneous population of antibodies only against a specific antigen epitope produced by a single B cell clone. That is, the individual antibodies that constitute the population are the same except for the possible naturally occurring mutations that may exist in small quantities. Monoclonal antibodies are highly specific for a single antigen. Monoclonal antibodies are advantageous because they can be obtained by culture of hybridoma cell lines and are essentially free from contamination by other immunoglobulins.

Monoclonal antibodies can be prepared by any of a variety of techniques known to those of ordinary skill in the art. For example, these methods may include the following steps: An immortal cell line capable of producing antibodies with the desired specificity is prepared. Such cell lines may be produced, for example, from spleen cells derived from immune animals. Spleen cells are then, immortalized, for example, by fusing with myeloma cell fusion partners (such as partners homologous to the animal being immunized). For example, membrane fusion promoters (such as polyethylene glycol or nonionic detergents) may be used to contacting spleen cells with myeloma cells for several minutes, and then plated at low density on selective medium that supports growth of hybrid cells but does not support growth of myeloma cells. After enough time, hybrid colonies are observed. Individual colonies are selected and tested for antigen binding activity. Hybridomas with high reactivity and specificity are preferred. Monoclonal antibodies may be isolated from the supernatant of the culture where hybridoma colonies grow. Contaminants may be removed from antibodies by conventional techniques such as chromatography, gel filtration, precipitation or extraction. For example, antibodies may be purified by chromatography with immobilized Protein G or Protein A using standard techniques.

Naturally occurring antibodies are produced by heavy chain only or by assembly of heavy and light chains. Each heavy chain consists of four domains: a variable region (VH), CH1, CH2 and CH3. Each light chain consists of a variable region (VL) and a constant region (CL). In the presence of heavy and light chains, the light chain is paired with a homologous heavy chain Fab fragment containing the VH and CH1 domains. The associated light and heavy chain Fab fragments are represented together as Fab fragments. The CH2 and CH3 domains represented together as the heavy chain Fc region are dimerized with the additional CH2 and CH3 domains from the second chain to form the Fc region. The Fc region is connected to the Fab fragment(s) via a flexible hinge region. The hinge region contains several disulfide bonds covalently connecting two heavy chain Fc regions together. In the Fab fragment, the light chain and the heavy chain are also connected by a disulfide bridge. However, the connectivity between IgG subclasses is different. The overall structure of full-length IgG is similar to a Y-shape, where the Fc region forms a base, while the two Fab fragments form arms and can be used to bind to an antigen.

The amino acid positions of all CDRs of the heavy and light chains of the antibodies of the present application are defined according to Kabat provided by Kabat, et al.(Sequences of Proteins of Immunological Interest, 5th ed., Public Health Service, National Institutes of Health, Bethesda, MD (1991)).

Complementarity determining regions (CDRs) are amino acid residues located within a variable region that are identified according to the Kabat definition, Chothia definition, AbM definition, contact definition and/or conformation definition or any other CDR determination method well known in the art. The CDRs of antibodies may be recognized as hypervariable regions originally defined by Kabat et al. see, for example, Kabat, et al., Sequences of Proteins of Immunological Interest, 5th ed., Public Health Service, National Institutes of Health, Bethesda, MD (1991). The locations of the CDRs may also be identified as the structural loop structure first described by Clothia et al. see, for example, Chothia et al., Nature 342:877-883, 1989. Other methods for identifying CDRs include the "AbM definition", a compromise between Kabat and Chothia and derived from that used by Oxford Molecular's AbM antibody modeling software. "Contact definition" is based on observed antigen contacts, such as MacCallim et al., J. Mol. Biol. 262:732-745, 1996. In the "conformation definition", the locations of CDRs may be identified as residues that make enthalpic contributions to antigen binding, see, for example, Makabe et al., Journal of Biological Chemistry, 283:1156- 1166, 2008.

As used herein, the amino acid sequences as set forth in SEQ ID NOs:1-60 are defined according to Kabat. Those skilled in the art will appreciate that CDR sequences determined by different definitions are not identical and the antibodies of the present application also encompass antibodies comprising CDRs defined by any of the Chothia definition, AbM definition, contact definition and conformation definition.

As used herein, the term "antigen-binding fragment" generally includes at least a portion (e.g., six CDRs) of the antigen-binding region, light chain and/or heavy chain variable regions of the parent antibody that retains at least some of the binding specificity of the parent antibody. Examples of antigen-binding fragments include, but are not limited to, Fab, Fab', F(ab')2, and Fv fragments.

Preferably, the antigen-binding fragment refers to the antigen-binding region, light and heavy chain variable regions or six CDRs of an antibody.

As used herein, the term "variant" refers to a sequence that may have a substitution, deletion, and/or addition of one or more amino acid residues compared to the sequence from which it derived. The biological activities of these variants are essentially unchanged compared to the sequences from which they are derived, such as allowing the antibodies to specifically bind to β-CTx when used in constituting the antibodies of the present application. The sequence from which the variant is derived maybe the full-length sequence, heavy chain, light chain, heavy chain variable region, light chain variable region, HCDR1, HCDR2, HCDR3, LCDR1, LCDR2 and LCDR3 of the antibody. Preferably, the amino acid substitution is a conservative substitution.

As used herein, the term "conservative substitution" refers to the substitution of residues in the current amino acid sequence with other amino acid residues with similar characteristics (such as charge, side chain size, hydrophobicity/hydrophilicity, main chain conformation and rigidity) such that changes in the type of amino acid residues occur without changing the biological activity of the protein. Exemplary conservative substitutions include mutual substitutions of amino acids in each of the following eight groups of: 1) alanine (A), glycine (G); 2) aspartic acid (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M) (see, for example, Creighton, Proteins (1984)).

In some embodiments, the variant after substitution, deletion or addition has at least 80%, at least 85%, at least 90%, at least 91%, at least 92%, at least 93%, at least 95%, at least 95%, at least 96%, at least 97%, at least 98% or at least 99% identity to the reference sequences.

As used herein, "percent (%) identity" relative to a reference polypeptide sequence refers to the percentage of amino acid residues in a variant sequence that are identical to the amino acid residues in the reference polypeptide sequence after aligning the sequences and introducing gaps if necessary to achieve the maximum percent sequence identity, and conservative substitutions are not considered identical for alignment purposes. Alignment to determine percent amino acid sequence identity can be achieved in a variety of ways within the skill of the art, for example, using publicly available computer software such as BLAST, BLAST-2, Clustal W, Megalign (DNASTAR) software or the FASTA package. Those skilled in the art can determine appropriate parameters for aligning sequences, including any algorithm needed to achieve maximum alignment over the full length of the sequences being compared. Alternatively, the sequence alignment computer program ALIGN-2 (Genentech, Inc.) may be used to produce a percent identity. The ALIGN-2 sequence alignment computer program was created by Genentech, Inc.

Preferably, substitution, deletion and addition are performed by adopting those substitutions, deletions and additions identified through alignment between the CDRs of different antibodies of the application. As described previously, alignments may be performed by BLAST, BLAST-2, Clustal W, Megalign (DNASTAR) software or the FASTA package, and the alignments may be, for example, pairwise alignments, or alignments between more CDRs. As an illustrative example, by alignment between the HCDR2 of the antibody b-CTx_R_rec_145 and the antibody b-CTx_R_rec_138, the mutation are substitutions of S6G, G7D and T9I.

In some embodiments, the antibody or the antigen-binding fragment thereof of the present application specifically binds to AH(betaD)G.

In some embodiments, the antibody or the antigen-binding fragment thereof of the present application specifically binds to AH(betaD)GG.

In some embodiments, the antibody or the antigen-binding fragment thereof of the present application specifically binds to AH(betaD)GGR.

In some embodiments, the antibody or the antigen-binding fragment thereof of the present application specifically binds to AH(betaD)GGR-COOH.

In other embodiments, the antibody or the antigen-binding fragment thereof of the present application specifically binds to more than one of AH(betaD)G, AH(betaD)GG, AH(betaD)GGR and AH(betaD)GGR-COOH. In one exemplary embodiment, the antibody or the antigen-binding fragment thereof of the present application specifically binds to AH(betaD)GG and AH (betaD)G.

The present application relates to: the hybridoma cell deposited with the Russian National Collection of Industrial Microorganisms (VKPM) (Research Centre "Kurchatov Institute" State Research Institute "Genetika" 1-st Dorozhniy pr., 1 Moscow 117545, Russian Federation) on March 28, 2024 under a deposit number of H-215, and the antibody b-CTx_M_hyb_015 produced by the hybridoma cell; antibodies comprising the heavy chain and light chain of the antibody produced by the hybridoma cell; antibodies comprising the heavy chain variable region and light chain variable region of the antibody produced by the hybridoma cell; and antibodies having 80%, at least 85%, at least 90%, at least 91%, at least 92%, at least 93%, at least 94%, at least 95%, at least 96%, at least 97%, at least 98% or at least 99% identity to the amino acid sequence of b-CTx_M_hyb_015. Preferably, the change caused by % identity does not occur at the CDRs or does not occur at the heavy chain variable region and the light chain variable region.

The present application relates to: the hybridoma cell deposited with the Russian National Collection of Industrial Microorganisms (VKPM) on March 28, 2024 under a deposit number of H-216; the antibody b-CTx_M_hyb_061 produced by the hybridoma cell; antibodies comprising the heavy chain and light chain of the antibody produced by the hybridoma cell; antibodies comprising the heavy chain variable region and light chain variable region of the antibody produced by the hybridoma cell; to antibodies having 80%, at least 85%, at least 90%, at least 91%, at least 92%, at least 93%, at least 94%, at least 95%, at least 96%, at least 97%, at least 98% or at least 99% identity to the amino acid sequence of b-CTx_M_hyb_061. Preferably, the change caused by % identity does not occur at the CDRs or does not occur at the heavy chain variable region and the light chain variable region.

The present application relates to: the hybridoma cell deposited with the Russian National Collection of Industrial Microorganisms (VKPM) on March 28, 2024 under a deposit number of H-217; the antibody b-CTx_M_hyb_063 produced by the hybridoma cell; antibodies comprising the heavy chain and light chain of the antibody produced by the hybridoma cell; antibodies comprising the heavy chain variable region and light chain variable region of the antibody produced by the hybridoma cell; and antibodies having 80%, at least 85%, at least 90%, at least 91%, at least 92%, at least 93%, at least 94%, at least 95%, at least 96%, at least 97%, at least 98% or at least 99% identity to the amino acid sequence of b-CTx_M_hyb_063. Preferably, the change caused by % identity does not occur at the CDRs or does not occur at the heavy chain variable region and the light chain variable region.

The present application relates to the cell deposited with VKPM on April 2, 2024, under a deposit number of B-14772, a plasmid carried by the cell, and a light chain produced by the plasmid; and relates to the cell deposited with VKPM on April 2, 2024 under a deposit number of B-14773, a plasmid carried by the cell, and a heavy chain produced by the plasmid. Further, the antibody b-CTx_R_rec_132 is provided, which comprises the light chain produced by the plasmid in B-14772 and the heavy chain produced by the plasmid in B-14773. Further provided is an antibody, which has 80%, at least 85%, at least 90%, at least 91%, at least 92%, at least 93%, at least 94%, at least 95%, at least 96%, at least 97%, at least 98% or at least 99% identity to the amino acid sequences of the heavy chain and light chain of b-CTx_R_rec_132. Preferably, the change caused by % identity does not occur at the CDRs or does not occur at the heavy chain variable region and the light chain variable region. Further provided is an antibody, which has the same heavy chain variable region and light chain variable region as b-CTx_R_rec_132.

The present application relates to the cell deposited with VKPM on April 2, 2024, under a deposit number of B-14774, a plasmid carried by the cell, and a light chain produced by the plasmid; and relates to the cell deposited with VKPM on April 2, 2024 under a deposit number of B-14775, a plasmid carried by the cell, and a heavy chain produced by the plasmid. Further, the antibody b-CTx_R_rec_134 is provided, which comprises the light chain produced by the plasmid in B-14774 and the heavy chain produced by the plasmid in B-14775. Further provided is an antibody, which has 80%, at least 85%, at least 90%, at least 91%, at least 92%, at least 93%, at least 94%, at least 95%, at least 96%, at least 97%, at least 98% or at least 99% identity to the amino acid sequences of the heavy chain and light chain of b-CTx_R_rec_134. Preferably, the change caused by % identity does not occur at the CDRs or does not occur at the heavy chain variable region and the light chain variable region. Further provided is an antibody, which has the same heavy chain variable region and light chain variable region as b-CTx_R_rec_134.

The present application relates to the cell deposited with VKPM on April 2, 2024, under a deposit number of B-14776, a plasmid carried by the cell, and a light chain produced by the plasmid; and relates to the cell deposited with VKPM on April 2, 2024 under a deposit number of B-14777, a plasmid carried by the cell, and a heavy chain produced by the plasmid. Further, the antibody b-CTx_R_rec_137 is provided, which comprises the light chain produced by the plasmid in B-14776 and the heavy chain produced by the plasmid in B-14777. Further provided is an antibody, which has 80%, at least 85%, at least 90%, at least 91%, at least 92%, at least 93%, at least 94%, at least 95%, at least 96%, at least 97%, at least 98% or at least 99% identity to the amino acid sequences of the heavy chain and light chain of b-CTx_R_rec_137. Preferably, the change caused by % identity does not occur at the CDRs or does not occur at the heavy chain variable region and the light chain variable region. Further provided is an antibody, which has the same heavy chain variable region and light chain variable region as b-CTx_R_rec_137.

The present application relates to the cell deposited with VKPM on April 2, 2024, under a deposit number of B-14778, a plasmid carried by the cell, and a light chain produced by the plasmid; and to the cell deposited with VKPM on April 2, 2024 under a deposit number of B-14779, a plasmid carried by the cell, and a heavy chain produced by the plasmid. Further, the antibody b-CTx_R_rec_138 is provided, which comprises the light chain produced by the plasmid in B-14778 and the heavy chain produced by the plasmid in B-14779. Further provided is an antibody, which has 80%, at least 85%, at least 90%, at least 91%, at least 92%, at least 93%, at least 94%, at least 95%, at least 96%, at least 97%, at least 98% or at least 99% identity to the amino acid sequences of the heavy chain and light chain of b-CTx_R_rec_138. Preferably, the change caused by % identity does not occur at the CDRs or does not occur at the heavy chain variable region and the light chain variable region. Further provided is an antibody, which has the same heavy chain variable region and light chain variable region as b-CTx_R_rec_138.

The present application relates to the cell deposited with VKPM on April 2, 2024, under a deposit number of B-14780, a plasmid carried by the cell, and a light chain produced by the plasmid; and relates to the cell deposited with VKPM on April 2, 2024 under a deposit number of B-14781, a plasmid carried by the cell, and a heavy chain produced by the plasmid. Further, the antibody b-CTx_R_rec_145 is provided, which comprises the light chain produced by the plasmid in B-14780 and the heavy chain produced by the plasmid in B-14781. Further provided is an antibody, which has 80%, at least 85%, at least 90%, at least 91%, at least 92%, at least 93%, at least 94%, at least 95%, at least 96%, at least 97%, at least 98% or at least 99% identity to the amino acid sequences of the heavy chain and light chain of b-CTx_R_rec_145. Preferably, the change caused by % identity does not occur at the CDRs or does not occur at the heavy chain variable region and the light chain variable region. Further provided is an antibody, which has the same heavy chain variable region and light chain variable region as b-CTx_R_rec_145.

The present application relates to the cell deposited with VKPM on April 2, 2024, under a deposit number of B-14782, a plasmid carried by the cell, and a light chain produced by the plasmid; and relates to the cell deposited with VKPM on April 2, 2024 under a deposit number of B-14783, a plasmid carried by the cell, and a heavy chain produced by the plasmid. Further, the antibody b-CTx_R_rec_150 is provided, which comprises the light chain produced by the plasmid in B-14782 and the heavy chain produced by the plasmid in B-14783. Further provided is an antibody, which has 80%, at least 85%, at least 90%, at least 91%, at least 92%, at least 93%, at least 94%, at least 95%, at least 96%, at least 97%, at least 98% or at least 99% identity to the amino acid sequences of the heavy chain and light chain of b-CTx_R_rec_150. Preferably, the change caused by % identity does not occur at the CDRs or does not occur at the heavy chain variable region and the light chain variable region. Further provided is an antibody, which has the same heavy chain variable region and light chain variable region as b-CTx_R_rec_150.

The present application relates to the cell deposited with VKPM on April 2, 2024, under a deposit number of B-14784, a plasmid carried by the cell, and a light chain produced by the plasmid; and relates to the cell deposited with VKPM on April 2, 2024 under a deposit number of B-14785, and a heavy chain produced by the cell. Further, the antibody b-CTx_R_rec_191 is provided, which comprises the light chain produced by the plasmid in B-14784 and the heavy chain produced by the plasmid in B-14785. Further provided is an antibody, which has 80%, at least 85%, at least 90%, at least 91%, at least 92%, at least 93%, at least 94%, at least 95%, at least 96%, at least 97%, at least 98% or at least 99% identity to the amino acid sequences of the heavy chain and light chain of b-CTx_R_rec_191. Preferably, the change caused by % identity does not occur at the CDRs or does not occur at the heavy chain variable region and the light chain variable region. Further provided is an antibody, which has the same heavy chain variable region and light chain variable region as b-CTx_R_rec_191.

In some embodiments, the KD of the antibodies of the present application is at least less than $10^{-9}$ M, especially $10^{-10}$ M.

As used herein, the term "nucleic acid molecule" or "polynucleotide" includes any compound and/or substance that comprises a polymer of nucleotides. Each nucleotide consists of a base, especially a purine or pyrimidine base (i.e., cytosine (C), guanine (G), adenine (A), thymine (T) or uracil (U)), a sugar (i.e., deoxyribose or ribose), and a phosphate group. Generally, a nucleic acid molecule is described by a sequence of bases, where the bases represent the primary structure (linear structure) of the nucleic acid molecule. The sequence of bases is usually expressed as being from 5' to 3'. As used herein, the term nucleic acid molecule includes deoxyribonucleic acid (DNA), including, for example, complementary DNA(cDNA) and genomic DNA; ribonucleic acid (RNA), especially messenger RNA(mRNA), synthetic forms of DNA or RNA, and mixed polymers containing two or more of these molecules. Nucleic acid molecules may be linear or circular. In addition, the term "nucleic acid molecule" includes sense and/or antisense strands, as well as single and double stranded forms. In addition, the nucleic acid molecules described herein may comprise naturally occurring or non-naturally occurring nucleotides. Examples of non-naturally occurring nucleotides include modified nucleotide bases with derivatized sugar or phosphate backbone bonds or chemically modified residues. Nucleic acid molecules also include DNA and RNA molecules suitable as vectors for direct expression of the antibodies of the present application in vitro and/or in vivo, for example, in a host or patient. Such DNA (e.g., cDNA) or RNA (e.g., mRNA) carriers may be unmodified or modified. For example, mRNA can be chemically modified to enhance the stability of the RNA vector and/or expression of the encoding molecule so that the mRNA can be injected into a subject to produce antibodies in the subject.

In a specific embodiment, the nucleic acid molecule of the present application encodes the antibody or the antigen-binding fragment thereof of the present application.

In a specific embodiment, the nucleic acid molecule of the present application encodes HCDR and LCDR. For example, the sequences of the nucleic acid molecules are as set forth in SEQ ID NOs: 61-66, SEQ ID NOs: 67-72, SEQ ID NOs: 73-78, SEQ ID NOs: 79-84, SEQ ID NOs: 85-90, SEQ ID NOs: 91-96, SEQ ID NOs: 97-102, SEQ ID NOs: 103-108, SEQ ID NOs: 109-114 or SEQ ID NOs: 115-120.

An "isolated" nucleic acid molecule refers to a nucleic acid molecule that has been separated from components of its natural environment. The isolated nucleic acid molecules include nucleic acid molecules contained in cells. The cells typically contain nucleic acid molecules but the nucleic acid molecules are present extra chromosomally or at chromosomal locations that are different from their natural chromosomal locations.

The "isolated nucleic acid molecule" encoding an antibody refers to one or more nucleic acid molecules encoding heavy and light chains (or antigen-binding fragments) of an antibody, including such nucleic acid molecule(s) in a single vector or separate vectors, and such nucleic acid molecule(s) present at one or more locations in a host cell.

As used herein, the term "vector" refers to a nucleic acid molecule capable of proliferating another nucleic acid to which it is attached. Vectors include vectors that are self-replicating nucleic acid structures and vectors that are incorporated into the genome of the host cell into which they have been introduced. Certain vectors are capable of directing the expression of the nucleic acid molecules to which they are operably linked. Such vectors are referred to herein as "expression vectors".

The terms "host cell", "host cell line" and "host cell culture" are used interchangeably and refer to cells into which foreign nucleic acid has been introduced, including the descendants of such cells. Host cells include "transformants" and "transformed cells", which include the primary transformed cells and the progeny derived therefrom, regardless of the number of passages. The progeny may not be identical in nucleic acid content to the parent cell, but may contain mutations. Included herein are mutant progeny with the same function or biological activity as screened or selected in the originally transformed cells.

In the present application, the expressions "first", "second", "third", etc. are used for descriptive purposes only to distinguish defined substances, and do not define order or priority in any way.

The kits of the present application may take a variety of forms, such as test kits for various reagents required for immunoassay kits, microfluidic chips, etc., and the kits may be manufactured according to standard procedures known to those skilled in the art.

The kit of the present application may comprise containers, solid phase carriers for immunoassay, a first antibody, a second antibody, instructions for use, buffers and/or other materials, structures and/or reagents needed for diagnosis/detection as needed.

The kit of the present application includes the antibody of the present application and may be present in a manner conventional in the art. In some embodiments, the antibody of the present application is present in a container in a dissolved or dried form, or coated on a solid carrier (such as membrane, plate, beads (such as magnetic beads and SA magnetic beads), and microspheres (such as carboxyl microspheres, amino microspheres, chloromethyl microspheres, and physically adsorbed microspheres)). In this case, the antibody of the present application may be referred to as a capture antibody.

Other reagents required for diagnosis/detection in the kits of the present application include, but are not limited to, other anti-$\beta$-CTx antibodies in addition to the antibodies of the present application, anti-rabbit antibodies, anti-mouse antibodies, etc. The above-mentioned other reactants may exist in a manner conventional in the art, for example, in a dissolved or dried form in a container, coated on a solid support, and in a dissolved or dried form in a chamber of the chip, but the present application is not limited thereto.

In specific embodiments, the components in the kit of the present application may exist independently. Depending on the different assay methods, the kit, for example, exists in the form of a first reagent and a second reagent; or exists in the form of a first reagent, a second reagent and a third reagent.

In specific embodiments, the measurement procedures of the kit are described in the instructions for use.

In some embodiments, the kit of the present application further includes a $\beta$-CTx standard. In one exemplary embodiment, the standard may be serially diluted. In specific embodiments, $\beta$-CTx antigen may be diluted to concentration points of 0 ng/mL, 0.07 ng/mL, 0.18 ng/mL, 0.32 ng/mL, 0.48 ng/mL, 0.82 ng/mL, 1.34 ng/mL, 2.81 ng/mL, 3.86 ng/mL, 5.62 ng/mL, and 6.57 ng/mL.

Other materials required for testing in the kit of the present application include, but are not limited to, materials for sampling, materials for conducting controls, and/or materials for observing the testing process or results.

Other reagents required for testing in the kit of the present application include, but are not limited to, detergents, color developers and/or terminating agents.

In some embodiments, the antibodies in the kit of the present application are labelled with a detectable label. Any label and labeling method known to those skilled in the art may be used. Commonly used labels may include enzymes (such as horseradish peroxidase, $\beta$-galactosidase, alkaline phosphatase), radioactive isotopes (such as $^{32}$P or $^{125}$I), biotin, digoxin, colloidal metals (such as colloidal gold), fluorescent dyes (such as fluorescein, rhodamine, Texas Red), chemiluminescent compounds or bioluminescent compounds (such as dioxetane, acridinium ester, luminol, isoluminol, acridinium, tripyridinium ruthenium). Any labeling step well known in the art may be used, such as covalent coupling of an enzyme or biotin group, iodination, phosphorylation, biotinylation. In this case, the antibody of the present application may be referred to as a detection antibody.

In some embodiments, one or more of the other reactants required for testing may also be labelled with a detectable label.

In some embodiments, the kit of the present application is an immunoassay kit based on a sandwich method, an indirect method, or a competitive method.

In addition, among the current mainstream $\beta$-CTx testing methods, high freshness of test samples is required for $\beta$-CTx, and the test needs to be completed within 4-8 h after sample collection. This requirement is not conducive to the operating habits of laboratory doctors and the requirements for retest. In order to meet the operating habits of laboratory doctors and the requirements for retest, the following improvements are necessary:

1. For serum samples, the storage time of serum at room temperature or 2-8 °C needs to be extended to at least 24 h to better serve testing and clinical applications. Therefore, the prior art methods may not meet this specific testing needs.
2. For heparin plasma or EDTA plasma samples, the storage time of serum at room temperature or 2-8 °C also needs to be extended to at least 24 h to better serve testing and clinical applications. Therefore, the prior art methods may not meet this specific testing needs.
3. Consistency between test results needs to be maintained regardless of serum or plasma samples.

To this end, the present application provides a kit comprising a first antibody and a second antibody, and

the first antibody comprises HCDR1, HCDR2, HCDR3, LCDR1, LCDR2 and LCDR3 having sequences as set forth in SEQ ID NOs: 13-18, respectively; and the second antibody comprises HCDR1, HCDR2, HCDR3, LCDR1, LCDR2 and LCDR3 having sequences as set forth in SEQ ID NOs: 37-42, respectively;
the first antibody comprises HCDR1, HCDR2, HCDR3, LCDR1, LCDR2 and LCDR3 having sequences as set forth in SEQ ID NOs: 49-54, respectively; and the second antibody comprises HCDR1, HCDR2, HCDR3, LCDR1, LCDR2 and LCDR3 having sequences as set forth in SEQ ID NOs: 31-36, respectively;
the first antibody comprises HCDR1, HCDR2, HCDR3, LCDR1, LCDR2 and LCDR3 having sequences as set forth in SEQ ID NOs: 1-6, respectively; and the second antibody comprises HCDR1, HCDR2, HCDR3, LCDR1, LCDR2 and LCDR3 having sequences as set forth in SEQ ID NOs: 7-12, respectively;
the first antibody comprises HCDR1, HCDR2, HCDR3, LCDR1, LCDR2 and LCDR3 having sequences as set forth in SEQ ID NOs: 55-60, respectively; and the second antibody comprises HCDR1, HCDR2, HCDR3, LCDR1, LCDR2 and LCDR3 having sequences as set forth in SEQ ID NOs: 7-12, respectively;
the first antibody comprises HCDR1, HCDR2, HCDR3, LCDR1, LCDR2 and LCDR3 having sequences as set forth in SEQ ID NOs: 19-24, respectively; and the second antibody comprises HCDR1, HCDR2, HCDR3, LCDR1, LCDR2 and LCDR3 having sequences as set forth in SEQ ID NOs: 1-6, respectively; or
the first antibody comprises HCDR1, HCDR2, HCDR3, LCDR1, LCDR2 and LCDR3 having sequences as set forth in SEQ ID NOs: 43-48, respectively; and the second antibody comprises HCDR1, HCDR2, HCDR3, LCDR1, LCDR2 and LCDR3 having sequences as set forth in SEQ ID NOs: 25-30, respectively.

The present application also includes a method for detecting the presence of lipoprotein (a) in a subject, the method comprising: contacting a biological sample from the subject with at least one antibody or antigen-binding fragment thereof of the present application to determine the presence, in the biological sample, of a molecule that is naturally present in the sample in a soluble form and has an antigenic determinant that is reactive with the at least one antibody or antigen-binding fragment thereof, the contacting being carried out under conditions and for a time sufficient to detect binding of the antibody or antigen-binding fragment thereof to the antigenic determinant.

In one embodiment, the antibody or the antigen binding fragment thereof is detectably labeled. In another embodiment, the antibody or the antigen-binding fragment thereof is not detectably labeled, and wherein the detection of binding of the antibody or the antigen-binding fragment thereof to an antigenic determinant is indirect.

Immunoassay testing methods include but are not limited to autoradiography, turbidimetry, fluorescence microscopy, direct and indirect enzymatic reactions, radioisotope methods or non-radioisotope methods, etc. These methods specifically include, immunoturbidimetry, latex-enhanced immunoturbidimetry, Western blotting, overlay assays, RIA (radioimmunoassay) and IRMA (immunoradioimmunoassay), GIA (colloidal gold immunoassay), EIA (enzyme immunoassay), ELISA (enzyme-linked immunosorbent assay), FIA (fluorescent immunoassay), and CLIA (chemiluminescent immunoassay).

Osteoporosis is a systemic bone disease characterized by low bone mass, destruction of bone microstructure, increased bone fragility, and susceptibility to fractures. Its clinical manifestations include bone pain, spinal deformation, fractures, back curvature, etc. The antibody or the antigen-binding fragment thereof or the kit described herein are used for diagnosing osteoporosis.

In one aspect, provided is use of the antibody or the antigen-binding fragment thereof of the present application, the kit of the present application, the antibody combination of the present application in the preparation of a kit for diagnosing osteoporosis.

In one aspect, provided is use of the antibody or the antigen-binding fragment thereof of the application, the kit of the present application, the antibody combination of the application in the preparation of a kit for detecting β-CTx.

In one aspect, the application provides a composition comprising one or more of the antibodies of the present application.

In some embodiments, the composition of the present application further comprises reagents that detect one or more of the group consisting of: osteocalcin, vitamin D, P1NP and PTH.

In some embodiments, the LOD of the kit, the antibody or the binding fragment of the antibody, or the composition, described herein, for detecting β-CTx is less than 11 pg/ml, less than 10 pg/ml, less than 9 pg/ml, less than 8 pg/ml, less than 7 pg/ml, less than 6 pg/ml, less than 5 pg/ml, less than 4 pg/ml, or less than 3.5 pg/ml.

In some embodiments, the cross-reactivity of the kit or the antibody or the antibody-binding fragment thereof, or the composition, described herein, for detecting β-CTx with α-CTx is less than or equal to 0.20%, less than or equal to 0.15%, less than or equal to 0.10%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, or less than or equal to 0.01%.

The kit, or the antibody or the antibody-binding fragment thereof, or the composition, described herein, is used for testing a serum sample or a plasma sample.

As used herein, the serum sample may be, for example, serum stored at 20~25 °C for up to 4 h, 6 h, 8 h,12 h or 16 h; or, for example, serum stored at 20~25 °C for more than 4 h, more than 6 h, more than 8 h, or more than 12 h. The serum sample may be, for example, serum stored at 2~8 °C for up to 4 h, 6 h, 8 h, 12 h, 24 h, or 2 days; or, for example, serum stored at 2~8 °C for more than 4 h, more than 6 h, more than 8 h, more than 10 h, more than 12 h, more than 16 h, or more than 20 h.

As used herein, the plasma sample may be heparin plasma or EDTA plasma.

The heparin plasma may be, for example, plasma stored at 20~25 °C for up to 4 h, 6 h, 8 h,12 h or 16 h; or, for example, plasma stored at 20~25 °C for more than 4 h, more than 6 h, more than 8 h, or more than 12 h. The heparin plasma may be, for example, plasma stored at 2~8 °C for up to 4 h, 6 h, 8 h, 12 h, 24 h, or 2 days; or, for example, plasma stored at 2~8 °C for more than 4 h, more than 6 h, more than 8 h, more than 10 h, more than 12 h, more than 16 h, or more than 20 h.

EDTA plasma may be, for example, plasma stored at 20~25 °C for up to 4 h, 6 h, 8 h, 12 h, 16 h, 24 h or 2 days; or, for example, plasma stored at 20~25 °C for more than 4 h, more than 6 h, more than 8 h, more than 12 h, more than 16 h, or more than 24 h. The EDTA plasma may be, for example, plasma stored at 2~8 °C for 1 day, 2 days, 4 days, 8 days or 10 days; or, for example, plasma stored at 2~8 °C for more than 2 days, more than 4 days or more than 8 days.

The implementation of the present application will be described in detail below in conjunction with examples. If specific conditions are not specified in the examples, routine conditions or conditions suggested by the manufacturer will be followed. The reagents or instruments used without indicating manufacturers are all conventional products that can be purchased commercially.

**Example 1 Generation of monoclonal antibodies specific for human Type I collagen β-C-terminal telopeptide (β-CTx)**

Recombinant human Type I collagen β-C-terminal telopeptide (β-CTx) was expressed in E. Coli and further purified. The resulting purified protein solution was concentrated, stored at -80 °C or below, and its purity was confirmed by SDS-PAGE and molecular sieve chromatography (SEC).

Immunization of mice was performed using β-CTx. The hybridoma cells obtained post-immunization were subsequently screened, to obtain hybridoma cells producing human Type I collagen β-C-terminal telopeptide (β-CTx).

Three mouse hybridomas were selected and deposited with VKPM.

The hybridoma cell line that produces b-CTx_M_hyb_015 was deposited with VKPM on March 28, 2024 under a deposit number of H-215;

the hybridoma cell line that produces b-CTx_M_hyb_061 was deposited with VKPM on March 28, 2024 under a deposit number of H-216;

and the hybridoma cell line that produces b-CTx_M_hyb_063 was deposited with VKPM on March 28, 2024 under a deposit number of H-217.

In addition, 7 rabbit hybridomas were obtained by a similar method.

**Example 2 Sequencing of mouse-derived antibodies**

Total RNA was extracted from selected hybridoma cells using the Quick-RNA Microprep Kit (#1050-51, Zymo Research, USA), and the antibodies produced were sequenced according to the instructions for the kit. The sequencing results of HCDRs and LCDRs are listed in Tables 1 and 2, respectively.

Table 1

| Name of Antibody | | Heavy chain | | |
|---|---|---|---|---|
| | | CDR1 | CDR2 | CDR3 |
| b-CTx_M_hy b_015 | nt | AGCACTTTTG GTATGGGTGT GAGC | CACATTTACTGGGATGATGT CAAGCGCTATAATCCATCCC TGAAGAGC | GCTCGAACCGCTGGGG CCTGGTTTGCTTTC |
| | aa | STFGMGVS | HIYWDDVKRYNPSLKS | ARTAGAWFAF |
| b-CTx_M_hy b_061 | nt | AGCACTTATG GTATGGGTAT AGGC | CACATTTGGTGGGATGATGT CAAGCGCTATGATCCAGTCC TGAAGAGC | GCTCGCATACCTGGGG ATGGTTCCCACAGTGA CTAC |
| | aa | STYGMGIG | HIWWDDVKRYDPVLKS | ARIPGDGSHSDY |
| b-CTx_M_hy b_063 | nt | AGCACTTCTG GTATGGGTAT AGGC | CACATTTGGTGGGATGATGT CAAGCGCTATGATCCAGCCC TGAAGAGC | GCTCGAATACCTGGGG ATGGTTCCCACTTTGAC TAC |
| | aa | STSGMGIG | HIWWDDVKRYDPALKS | ARIPGDGSHFDY |

Table 2

| Name of Antibody | | Light chain | | |
|---|---|---|---|---|
| | | CDR1 | CDR2 | CDR3 |
| b-CTx_M_ hyb_015 (kappa) | nt | AGTGCCAGGTCAACTGTAA ATTACATGCAC | GACACATCCA AACTGGCTTCT | CAGCAGTGGACTAGT AATTTGCTCACG |
| | aa | SARSTVNYMH | DTSKLAS | QQWTSNLLT |
| b-CTx_M_ hyb_061 (kappa) | nt | AGATCTACTCAGAGCCTTG TACATAGTAATGGAATCACC TATTTAGAG | AGAATTTCCAA CCGATTTTCT | TTTCAAGGTTCACATG GTCCGTACACG |
| | aa | RSTQSLVHSNGITYLE | RISNRFS | FQGSHGPYT |
| b-CTx_M_ hyb_063 (kappa) | nt | AGATCTAGTCAGAGCCTTG TACATAGTAATGGAATCACC TATTTAGAG | AGAGTTTCCA ACCGATTTTCT | TTTCAAGGTTCACATG GTCCGTACACG |
| | aa | RSSQSLVHSNGITYLE | RVSNRFS | FQGSHGPYT |

**Example 3 Sequencing of rabbit-derived antibodies**

Total RNA was extracted from approximately 500 hybridoma cells using Norgen Single Cell Purification Kit (#51800, Norgen Biotek, Canada), and the antibodies produced were sequenced according to the instructions for the kit. The sequencing results of HCDRs and LCDRs are listed in Tables 3 and 4, respectively.

Table 3

| Name of Antibody | | Heavy chain | | |
|---|---|---|---|---|
| | | CDR1 | CDR2 | CDR3 |
| b-CTx_R_rec_132 | nt | AGTAGCGCTGCAATAAAC | TACATTTATGCTGGTAGTGCTAGCACATACTACGCGACCTGGGCGAAAGGC | GCCAGAAGTTTGAGTGGTGGTGATCGGAACTTG |
| | aa | SSAAIN | YIYAGSASTYYATWAKG | ARSLSGGDRNL |
| b-CTx_R_rec_134 | nt | AGTAGCGCTGCAATAAAT | TACATTTATGCTGGTAGTGGTAGCACATACTACGCGAGCTGGGCGAAAGGC | GCCAGAAGTTTGAGTGGCGGTGATCGGAACTTG |
| | aa | SSAAIN | YIYAGSGSTYYASWAKG | ARSLSGGDRNL |
| b-CTx_R_rec_137 | nt | AGTAGCGCTGCAATAAAT | TACATTTATGCTGGTAGTGGTAGCACATACTACGCGAGCTGGGCGAAAGGC | GCCAGAAGTTTGAGTGGCGGTGATCGGAACTTG |
| | aa | SSAAIN | YIYAGSGSTYYASWAKG | ARSLSGGDRNL |
| b-CTx_R_rec_138 | nt | AGTAGCGCTGCAATAAAC | TACATTTATGCTGGTGATGATAGTATATACTACGCGAGCTGGGCGAAAGGC | GCCAGAAGTTTGAGTGGTGGTGATCGGAACTTG |
| | aa | SSAAIN | YIYAGDDSIYYASWAKG | ARSLSGGDRNL |
| b-CTx_R_rec_145 | nt | AGTAGCGCTGCAATAAAC | TACATTTATGCTGGTAGTGGTAGCACATACTACGCGAGCTGGGCGAAAGGC | GTCAGAAGTTTAAGTGGTGGTGATCGGAACTTG |
| | aa | SSAAIN | YIYAGSGSTYYASWAKG | VRSLSGGDRNL |
| b-CTx_R_rec_150 | nt | AGTAAATATGGAGTGAAC | TGGATTACTAGTGATGGTATCACATTCTACGCGAGCTGGGCGAAGGC | ACCAGAAGTATACTTAGTGCTTGGGATAACATC |
| | aa | SKYGVN | WITSDGITFYASWAKG | TRSILSAWDNI |
| b-CTx_R_rec_191 | nt | AGTAGCTACTACGTGAGC | GGCATTAATACTGGTGGTGTCGCATACTACGCGACCTGGGCAAAAGGC | GCCAGACATCCTGGTGGTGATGCTGGTACCGACTTG |
| | aa | SSYYVS | GINTGGVAYYATWAKG | ARHPGGDAGTDL |

Table 4

| Name of Antibody | | Light chain (with all of Kappa) | | |
|---|---|---|---|---|
| | | CDR1 | CDR2 | CDR3 |
| b-CTx_R_rec_132 | nt | CAGTCCAGTGAGAGTGTTACTAAGAACAACTACTTAGCC | CTCCTGATCTACGAAGCATCCAAACTGGCA | CAAGGCGGTTATAGTAGGAATAATGATATGACT |
| kappa | aa | QSSESVTKNNYLA | LLIYEASKLA | QGGYSRNNDMT |
| b-CTx_R_rec_134 | nt | CAGTCCAGTGAGAATGTTGCTAAGAACAACTACTTAGCC | CTCCTGATCTACGAAGCATCCAAACTGGCA | CAAGGCGGTTATAATAGGAATAATGATATGACT |
| kappa | aa | QSSENVAKNNYLA | LLIYEASKLA | QGGYNRNNDMT |

| b-CTx_R_rec_137 | nt | CAGTCCAGTGAGAGTGTTTCTAAGAACAACTACTTAGCC | CTCCTGATCTACGAAGCATCCAAACTGGCA | CAAGGCGGTTATAGTAGGAATAATGATATGACT |
| kappa | aa | QSSESVSKNNYLA | LLIYEASKLA | QGGYSRNNDMT |
| b-CTx_R_rec_138 | nt | CAGTCCAGTGAGAGTGTTTCTAAGAACAACTACTTAGCC | CTCCTGATCTACGAAGCATCCAAACTGCCA | CAAGGCGGTTATAGTAGGAATAATGATATGACT |
| kappa | aa | QSSESVSKNNYLA | LLIYEASKLP | QGGYSRNNDMT |
| b-CTx_R_rec_145 | nt | CAGTCCAGTGAGAGTGTTTCTAAGAATGATTACTTAGCC | CTCCTGATCTACAGGGCTTCCACTCTGGCA | CAAGGCGGTTATAGTAGGAATAATGATATGACT |
| kappa | aa | QSSESVSKNDYLA | LLIYRASTLA | QGGYSRNNDMT |
| b-CTx_R_rec_150 | nt | CAGTCCAGTAAGAGTGTTTATAATGGGAACTGGTTATCC | CGCCTGATCTATTCTGCATCCACTCTGGCA | GCAGGCGGTTATAGTGGTGAGATTGATGAT |
| kappa | aa | QSSKSVYNGNWLS | RLIYSASTLA | AGGYSGEIDD |
| b-CTx_R_rec_191 | nt | CAGGCCAGTCAGAGCATTACTAAATACTTAGCC | CGCCTGATCTACAAGACATCCACTCTGGCA | CAAAACAATGTTCCTGGTAAAATTACTGACAGTTATGGGGCT |
| kappa | aa | QASQSITKYLA | RLIYKTSTLA | QNNVPGKITDSYGA |

## Example 4 Production and purification of antibodies

Hybridoma cells H-125, H-216 and H-217 were cultured in vitro, and antibodies b-CTx_M_hyb_015, b-CTx_M_hyb_061 and b-CTx_M_hyb_063 were isolated using conditioned medium for cell culture.

Rabbit antibodies b-CTx_R_rec_132, b-CTx_R_rec_134, b-CTx_R_rec_137, b-CTx_R_rec_138, b-CTx_R_rec_145, b-CTx_R_rec_150 and b-CTx_R_rec_191 were produced in recombinant forms. Plasmids encoding the amino acid sequences of the light chain and heavy chain of the recombinant antibody were obtained from the 7 rabbit hybridomas in Example 1, and competent cells carrying the plasmids were deposited at VKPM, with the following details:

cells introduced with the plasmid containing the insert encoding the amino acid sequence of the heavy chain of b-CTx_R_rec_132 was deposited at VKPM on April 2, 2024 under a deposit number of B-14773; cells introduced with the plasmid containing the insert encoding the amino acid sequence of the light chain of b-CTx_R_rec_132 was deposited at VKPM on April 2, 2024 under a deposit number of B-14772;

cells introduced with the plasmid containing the insert encoding the amino acid sequence of the heavy chain of b-CTx_R_rec_134 was deposited at VKPM on April 2, 2024 under a deposit number of B-14775; cells introduced with the plasmid containing the insert encoding the amino acid sequence of the light chain of b-CTx_R_rec_134 was deposited at VKPM on April 2, 2024 under a deposit number of B-14774;

cells introduced with the plasmid containing the insert encoding the amino acid sequence of the heavy chain of b-CTx_R_rec_137 was deposited at VKPM on April 2, 2024 under a deposit number of B-14777; cells introduced with the plasmid containing the insert encoding the amino acid sequence of the light chain of b-CTx_R_rec_137 was deposited at VKPM on April 2, 2024 under a deposit number of B-14776;

cells introduced with the plasmid containing the insert encoding the amino acid sequence of the heavy chain of b-CTx_R_rec_138 was deposited at VKPM on April 2, 2024 under a deposit number of B-14779; cells introduced with the plasmid containing the insert encoding the amino acid sequence of the light chain of b-CTx_R_rec_138 was deposited at VKPM on April 2, 2024 under a deposit number of B-14778;

cells introduced with the plasmid containing the insert encoding the amino acid sequence of the heavy chain of b-CTx_R_rec_145 was deposited at VKPM on April 2, 2024 under a deposit number of B-14781; cells introduced with the plasmid containing the insert encoding the amino acid sequence of the light chain of b-CTx_R_rec_145 was deposited at VKPM on April 2, 2024 under a deposit number of B-14780;

cells introduced with the plasmid containing the insert encoding the amino acid sequence of the heavy chain of b-CTx_R_rec_150 was deposited at VKPM on April 2, 2024 under a deposit number of B-14783; cells introduced with the plasmid containing the insert encoding the amino acid sequence of the light chain of b-CTx_R_rec_150 was deposited at VKPM on April 2, 2024 under a deposit number of B-14782;
cells introduced with the plasmid containing the insert encoding the amino acid sequence of the heavy chain of b-CTx_R_rec_191 was deposited at VKPM on April 2, 2024 under a deposit number of B-14785; cells introduced with the plasmid containing the insert encoding the amino acid sequence of the light chain of b-CTx_R_rec_191 was deposited at VKPM on April 2, 2024 under a deposit number of B-14784.

Mammalian cell line Expi293F was transfected with molecular gene constructs of recombinant antibodies.

For example, the light chain and heavy chain gene sequences are transferred into the open reading frames (ORF) of expression vectors by homologous recombination or restriction digestion respectively to form the "promoter-antibody light chain gene-terminator" and "promoter-antibody heavy chain gene-terminator" structures on the two expression vectors respectively. Then, by biological, physical or chemical methods, the two expression vectors (antibody light chain expression vector and heavy chain expression vector) were simultaneously transferred into the mammalian cell line Expi293F for expression. Finally, the two light chains and two heavy chains were reassembled into a complete antibody in the cell and secreted into the culture supernatant. Alternatively, the expression vector is modified to contain two open reading frames, and then the light chain and heavy chain gene sequences are inserted into the two open reading frames (ORF) of the expression vector respectively by homologous recombination or restriction digestion to form a "promoter-antibody light chain gene-terminator-vector sequence-promoter-antibody heavy chain gene-terminator" or a "promoter-antibody heavy chain gene-terminator-vector sequence-promoter-antibody light chain gene-terminator" structure. Then, by biological, physical or chemical methods, this expression vector containing both the light and heavy chains of the antibody was transferred into the mammalian cell line Expi293F for expression. Finally, the two light and two heavy chains were reassembled into a complete antibody in the cell and secreted into the culture supernatant.

Antibodies were purified from conditioned media using Protein A affinity chromatography. The chromatography matrix was obtained from GE Healthcare Life Sciences (Piscataway, NJ) and the purification process was performed according to the manufacturer's instructions. Purified monoclonal antibodies were stored in 50% ammonium sulfate as a suspension at 4 °C.

**Example 5 Determination of affinity of antibodies**

The affinity of all anti-β-CTx MAbs were determined by affinity biolayer interferometry (Octet Sartorius, Germany).

Affinity biolayer interferometry was performed according to the following protocol:

- Amine Reactive 2nd Generation (AR2G) Octet biosensor was hydrated in deionized water.
- The hydrated biosensor was activated with an aqueous solution of 1 mM MEDC (1-ethyl-3-[3-dimethylaminopropyl] carbodiimide hydrochloride) and 0.5 mM s-NHS (N-hydroxysulfosuccinimide) at 25°C for 5 min.
- The synthesized β-CTx peptide EKAH(beta-D)GGR was immobilized on the activated biosensor for 10 min at 25 °C.
- The biosensor with the β-CTx peptide immobilized was blocked with 1M ethanolamine (pH 8.5) at 25 °C for 5 min.

The antibody solutions (1, 2, 5 μg/ml) in PBS were applied to the sensor according to the following protocol:

- hydrating the sensor;

    - -binding the antibody to the sensor;

- cleaning the sensor;
- blocking the sensor;
- association (perform association for each concentration of antibody separately);
- dissociation;
- sensor regeneration;

The affinity constants of antibodies are shown in Table 5 below.

Table 5

| Name of MAb | KD (M) |
|---|---|
| b-CTx_M_hyb_015 | 5.3E-9 |
| b-CTx_M_hyb_061 | 4.1E-9 |

(continued)

| Name of MAb | KD (M) |
|---|---|
| b-CTx_M_hyb_063 | 3.4E-9 |
| b-CTx_R_rec_132 | 8.5E-10 |
| b-CTx_R_rec_134 | 1.1E-9 |
| b-CTx_R_rec_137 | 2.7E-9 |
| b-CTx_R_rec_138 | 2.0E-9 |
| b-CTx_R_rec_145 | 8.9E-10 |
| b-CTx_R_rec_150 | 1.1E-9 |
| b-CTx_R_rec_191 | 7.1E-9 |

It can be seen from Table 5 that all antibodies have good ability to bind to $\beta$-CTx, the KD of b-CTx_R_rec_145 reaches 8.9E-10, and the KD of b-CTx_R_rec_132 reaches 8.5E-10.

**Example 6 Development of chemiluminescent sandwich immunoassay**

Different paired combinations of all MAbs were tested using a sandwich immunoassay to find the combination with the best characteristics.

For chemiluminescent sandwich particle-based immunoassay, detection MAbs were labeled with alkaline phosphatase (cat# 03137031103, Roche Custom Biotech) and capture MAbs were labeled with biotin (Cat #PG82075, Thermo). The capture antibodies were incubated with streptavidin-labeled particles (MyOne T1 Dynabeads Streptavidin PMP Hydrophobic particles 100 mkm, Cat# 35604D, Thermo), and the antibody-labeled particles were used for detection in a solution of 0.25 mg/ml 50 mM Tris-HCl, pH 7.4, 150 mM NaCl, 2% BSA, 0.2% Tween-20, 0.05% ProClin 300, 0.09% $NaN_3$. The detection antibodies were used at a concentration of 0.5 $\mu$g/ml in a solution of 50 mM MES, pH6, 0.9% NaCl, 1% BSA, 0.05% Tween-20, 0.048% ProClin 300, 2 mM $MgCl_2$, 0.1 mM $ZnCl_2$. The antibody pairs used (capture-detection) are listed in Table 6.

Pooled human plasma samples with pre-measured $\beta$-CTx concentrations were employed as calibrators in the immunoassay. Concentrations of $\beta$-CTx in calibrator plasma samples were determined using the Roche beta-CrossLaps immunoassay on a Cobas 6000 automated analyzer (Roche Diagnostics GmbH). Pooled human plasma samples were serially diluted in a solution of 50 mM MES, pH 6, 0.9% NaCl, 1% BSA, 0.05% Tween-20, 0.048% ProClin 300, 2 mM $MgCl_2$, 0.1 mM $ZnCl_2$. 50 $\mu$L of calibrator plasma was mixed with 50 $\mu$L of particle suspension and 50 $\mu$L of detection MAb-phosphatase conjugate solution. A one-step immunoassay was performed in an automated analyzer Mindray CL-6000i according to the manufacturer's instructions. Chemiluminescence was detected using the substrate solution provided together with the analyzer. Chemiluminescence is expressed in relative light units (RLU).

Serum supplemented with 150 ng/mL $\alpha$-CTx was used to test the $\alpha$-CTx cross-reactivity of different antibody pairs. The cross-reactivity percentage was calculated from the measured concentration/supplemented concentration.

In addition, the limit of detection (LOD) for different antibody pairs was tested based on the following protocol: Limit of Blank (LOB) and LOD were determined according to suggestions of Clinical and Laboratory Standards Institute (CLSI) (Evaluation of Detection Capability for Clinical Laboratory Measurement Procedures; Approved Guideline-Second Edition; EP17-A Vol. 24 No. 34).

Twenty replicates of zero analytes (50 mM MES, pH 6, 0.9% NaCl, 1% BSA, 0.05% Tween-20, 0.048% ProClin 300, 2 mM $MgCl_2$, 0.1 mM $ZnCl_2$) were measured using the corresponding chemiluminescent sandwich immunoassay.

The calculation formulas are

$$LOB = 1.64*Mean;$$

$$LOD = LOB + 1.65*SD$$

Test results of cross-reactivity and LOD are listed in Table 6.

Table 6

| Capture MAb | Detection MAb | LOD pg/ml | Cross-reactivity with α-CTx |
|---|---|---|---|
| b-CTx_M_hyb_061 | b-CTx_R_rec_145 | 4.0 | 0.02% |
| b-CTx_M_hyb_063 | b-CTx_R_rec_145 | 4.1 | 0.01% |
| b-CTx_R_rec_145 | b-CTx_R_rec_138 | 3.3 | 0.02% |
| b-CTx_M_hyb_015 | b-CTx_R_rec_137 | 10.9 | 0.20% |
| b-CTx_M_hyb_015 | b-CTx_R_rec_191 | 10.0 | 0.14% |
| b-CTx_M_hyb_015 | b-CTx_R_rec_145 | 9.7 | 0.14% |
| b-CTx_M_hyb_061 | b-CTx_R_rec_132 | 7.0 | 0.03% |
| b-CTx_R_rec_132 | b-CTx_R_rec_138 | 6.4 | 0.01% |
| b-CTx_R_rec_132 | b-CTx_R_rec_145 | 6.1 | 0.02% |
| b-CTx_R_rec_134 | b-CTx_R_rec_138 | 6.6 | 0.03% |
| b-CTx_R_rec_134 | b-CTx_R_rec_145 | 6.3 | 0.00% |
| b-CTx_R_rec_137 | b-CTx_R_rec_138 | 5.3 | 0.02% |
| b-CTx_R_rec_137 | b-CTx_R_rec_145 | 4.6 | 0.03% |
| b-CTx_R_rec_191 | b-CTx_R_rec_138 | 6.0 | 0.06% |
| b-CTx_R_rec_191 | b-CTx_R_rec_145 | 5.8 | 0.01% |
| b-CTx_R_rec_150 | b-CTx_R_rec_132 | 7.2 | 0.01% |
| b-CTx_R_rec_150 | b-CTx_R_rec_137 | 7.1 | 0.00% |
| b-CTx_R_rec_191 | b-CTx_R_rec_132 | 6.2 | 0.03% |
| b-CTx_R_rec_191 | b-CTx_M_hyb_061 | 7.0 | 0.17% |
| b-CTx_M_hyb_061 | b-CTx_R_rec_134 | 7.1 | 0.03% |
| b-CTx_M_hyb_061 | b-CTx_R_rec_137 | 7.0 | 0.02% |
| b-CTx_M_hyb_063 | b-CTx_R_rec_137 | 7.3 | 0.06% |

It can be seen from Table 6 that the cross-reactivity percentage with α-CTx for all tested antibody pairs was 0.20% or below, and for some antibody pairs was even 0.05% or below. The LOD of all antibody pairs tested was 10.9 pg/ml or below, and for some antibody pairs was even as low as 5 pg/ml. The LOB and LOD of sandwich immunoassay using the combination of b-CTx_R_rec_145 and b-CTx_R_rec_138 were 2.0 and 3.3 pg/ml, respectively.

**Example 7 Antibody epitope mapping**

As shown in Table 7, the precise epitopes of all anti-β-CTx MAbs were determined by using extended or truncated peptides.

Enzyme-linked immunosorbent assay (ELISA) using β-CTx peptide analogues conjugated with ovalbumin as pre-adsorbed antigen was adopted for detecting β-CTx-specific antibodies. Each well was added with 2020 ng/0.05ml β-CTx peptide analogue-ovalbumin and incubated under RT conditions with gentle agitation. After 30 minutes of antigen adsorption, the plates were washed twice with PBS containing 0.1% detergent Tween 20 (PBST). The plates were then incubated with 0.05 ml of 1 μg/ml antibodies for 30 min and washed twice with PBST. HRP-labeled anti-mouse IgG or anti-rabbit IgG secondary polyclonal antibodies (0.05 ml per well, diluted with PBST at 1:1000) was added and incubated for 30 min to detect mouse or rabbit antibodies bound to pre-adsorbed antigen. The secondary antibodies were obtained from Sigma Chemicals, St. Louise, Mo. After incubation with the secondary antibody, the plates were washed six times with PBST, and then 0.05 ml of 3,3',5,5'-tetramethylbenzidine (TMB) peroxidase substrate solution containing 0.03% hydrogen peroxide was added. After incubation for 10 min, 0.05 ml of 1M phosphoric acid was added to stop the reaction, and the absorbance in the wells was measured at a wavelength of 450 nm. Absorbance was measured using a Labsystems Multiscan microplate reader (Labsystems, Finland).

Table 7

| Antibody | Ova-CEKAH-(be-taD)-GGR | EKAH-(be-taD)-GGRG GC-Ova | Ova-CEKAH-(be-taD)-GGRY YRA | Ova-CGGAH-(be-taD)-GGR | Ova-CEKAH-(be-taD)-GG | Ova-CEKAH-(betaD) G | Background value | Epitope |
|---|---|---|---|---|---|---|---|---|
| b-CTx_M_hyb_015 | 1.658 | 1.510 | 1.497 | 1.453 | 1.048 | 0.899 | 0.154 | AH(beta D)G |
| b-CTx_M_hyb_061 | 1.647 | 1.273 | 1.220 | 2.013 | 0.061 | 0.049 | 0.045 | AH(beta D)GGR |
| b-CTx_M_hyb_063 | 1.884 | 1.675 | 1.677 | 1.806 | 0.058 | 0.059 | 0.044 | AH(beta D)GGR |
| b-CTx_R_rec_132 | 1.400 | 0.412 | 0.467 | 1.299 | 0.139 | 0.121 | 0.045 | AH(beta D)G |
| b-CTx_R_rec_134 | 1.227 | 0.361 | 0.302 | 1.292 | 0.141 | 0.089 | 0.044 | AH(beta D)GG/ AH(beta D)G |
| b-CTx_R_rec_137 | 1.266 | 0.422 | 0.326 | 1.315 | 0.099 | 0.069 | 0.044 | AH(beta D)GG |
| b-CTx_R_rec_138 | 1.293 | 0.413 | 0.767 | 1.807 | 0.070 | 0.062 | 0.042 | AH(beta D)GGR |
| b-CTx_R_rec_145 | 1.335 | 0.812 | 0.733 | 1.519 | 0.521 | 0.185 | 0.044 | AH(beta D)G |
| b-CTx_R_rec_150 | 1.436 | 0.063 | 0.142 | 1.364 | 0.082 | 0.079 | 0.046 | AH(beta D)GGR-COOH |
| b-CTx_R_rec_191 | 0.975 | 0.045 | 0.089 | 1.738 | 0.073 | 0.079 | 0.043 | AH(beta D)GGR-COOH |

It can be seen from Table 7 that the epitopes of all anti-β-CTx antibodies have 4-6 amino acid residues in the β-CTx octapeptide. Antibody b-CTx_R_rec_134 binds to both epitopes AH(betaD)GG and AH(betaD)G. Antibodies b-CTx_R_rec_150 and b-CTx_R_rec_191 effectively recognize β-CTx in the presence of a C-terminal arginine residue with a free carboxyl group.

**Example 8 Assessment on sample stability**

8.1 Preparation of kits

Step 1: Preparation of reagent R1

Streptavidin and superparamagnetic particles were mixed in TBS buffer, and after sufficient reaction, the mixture was placed on a magnetic separator until the supernatant became clear. Then, the supernatant was removed, and the streptavidin-coated superparamagnetic particles were retained and washed three times with TBS buffer, and finally dissolved with R1 diluent to achieve the final superparamagnetic particle content of 0.05%, thus obtaining the reagent R1, which was refrigerated at 2~8 °C. The components of reagent R1 may be prepared by conventional methods.

Step 2: Preparation of reagent R2

A detection antibody was conjugated to alkaline phosphatase, wherein the molar ratio of the conjugate to the detection antibody was 15:1. Then, the conjugate was dissolved with R2 diluent to achieve the final concentration of 2 μg/mL, thus obtaining reagent R2, which was refrigerated at 2~8 °C. The components of reagent R2 may be prepared by conventional methods.

Step 3: Preparation of reagent R3

A capture antibody was conjugated to biotin, wherein the molar ratio of the conjugate to the capture antibody was 30:1. Then, the conjugate was dissolved with R3 diluent to achieve the final concentration of 1 μg/mL, thus obtaining reagent R3, which was refrigerated at 2~8 °C. The components of reagent R3 may be prepared by conventional methods.

Step 4: Preparation of β-CTx calibrators

β-CTx calibrators were prepared by diluting β-CTx antigen to concentration points of 0 ng/mL, 0.07 ng/mL, 0.18 ng/mL, 0.32 ng/mL, 0.48 ng/mL, 0.82 ng/mL, 1.34 ng/mL, 2.81 ng/mL, 3.86 ng/mL, 5.62 ng/mL, and 6.57 ng/mL.
Kits were prepared separately using the antibody pairs listed in Table 8 below for subsequent experiments.

Table 8

| Kit | Capture antibody | Detection antibody |
| --- | --- | --- |
| A | b-CTx_R_rec_132 | b-CTx_R_rec_191 |
| B | b-CTx_M_hyb_061 | b-CTx_R_rec_150 |
| C | b-CTx_R_rec_145 | b-CTx_R_rec_138 |
| D | b-CTx_M_hyb_063 | b-CTx_R_rec_138 |
| E | b-CTx_R_rec_134 | b-CTx_R_rec_145 |
| F | b-CTx_M_hyb_015 | b-CTx_R_rec_137 |

8.2 Measurement procedures of the β-CTx kit:

(1) The β-CTx kit (including reagent R1, reagent R2 and reagent R3) was loaded into the Mindray CL series automatic chemiluminescent immunoanalyzer.
(2) The sample volume required by the system for each measurement was 50 μL, and the total measurement time per report was 32 min.
(3) The specific sample loading process of the detection system was as follows: 50 μL of sample and 50 μL of reagent

R3 was added to a reaction tube. After incubation, the β-CTx in the sample bound to the anti-β-CTx antibody in R3. Then, 50 μL of reagent R1 and 50 μL of reagent R2 were added at the same time. After incubation, the labeled anti-β-CTx antibody bound to another site of β-CTx in the sample to form a sandwich complex. After the reaction was completed, the magnetic beads were immobilized using a magnetic field and unbound substances were removed through washing.

The reaction substrate or excitation liquid was then added to the reaction tube to generate an optical signal, and the number of photons generated was measured. The number of photons generated was in direct proportion to the concentration of β-CTx in the sample.

(4) The matched β-CTx calibrator was used for calibration test. Based on the calibration data, the system software mathematically fitted the optical signal to the concentration by using a weighted four-parameter logarithmic curve (4PLC), and the final result was given in a concentration form of ng/mL.

8.3 The kits prepared using b-CTx_R_rec_132 and b-CTx_R_rec_191 were used to detect different samples according to the measurement procedures of 8.2. The results are shown in Table 9.

Table 9

| Serum-sample stability (20~25 °C) | | | |
|---|---|---|---|
| b-CTx_R_rec_132 and b-CTx_R_rec_191 | 0 h concentration-ng/mL | 12 h concentration-ng/mL | Variation |
| Serum sample-1 | 0.637 | 0.608 | -4.6% |
| Serum sample-2 | 0.227 | 0.217 | -4.3% |
| Serum sample-3 | 0.392 | 0.385 | -1.8% |

| Serum-sample stability (2~8 °C) | | | |
|---|---|---|---|
| b-CTx_R_rec_132 and b-CTx_R_rec_191 | 0 h concentration-ng/mL | 24 h concentration-ng/mL | Variation |
| Serum sample-11 | 0.613 | 0.634 | 3.3% |
| Serum sample-12 | 0.479 | 0.487 | 1.5% |
| Serum sample-13 | 0.913 | 0.834 | -8.7% |

| | | | |
|---|---|---|---|
| Serum sample-4 | 0.129 | 0.121 | -5.6% |
| Serum sample-5 | 0.672 | 0.620 | -7.9% |
| Serum sample-6 | 0.186 | 0.175 | -5.8% |
| Serum sample-7 | 0.475 | 0.453 | -4.7% |
| Serum sample-8 | 0.448 | 0.426 | -4.9% |
| Serum sample-9 | 0.368 | 0.352 | -4.2% |
| Serum sample-10 | 0.339 | 0.325 | -4.0% |
| | | Mean variation | -4.8% |

| | | | |
|---|---|---|---|
| Serum sample-14 | 0.324 | 0.332 | 2.4% |
| Serum sample-15 | 0.648 | 0.621 | -4.2% |
| Serum sample-16 | 0.148 | 0.149 | 0.7% |
| Serum sample-17 | 0.353 | 0.326 | -7.6% |
| Serum sample-18 | 0.215 | 0.208 | -3.3% |
| Serum sample-19 | 0.530 | 0.522 | -1.6% |
| Serum sample-20 | 0.384 | 0.375 | -2.5% |
| | | Mean variation | -2.0% |

| Heparin lithium-sample stability (20~25 °C) | | | |
|---|---|---|---|
| b-CTx_R_rec_132 and b-CTx_R_rec_191 | 0 h concentration-ng/mL | 12 h concentration-ng/mL | Variation |
| Heparin lithium sample-1 | 0.660 | 0.674 | 2.1% |
| Heparin lithium sample-2 | 0.249 | 0.258 | 3.5% |
| Heparin lithium sample-3 | 0.423 | 0.398 | -5.9% |
| Heparin lithium sample-4 | 0.136 | 0.127 | -6.5% |
| Heparin lithium sample-5 | 0.735 | 0.679 | -7.7% |
| Heparin lithium sample-6 | 0.182 | 0.170 | -6.8% |
| Heparin lithium sample-7 | 0.493 | 0.477 | -3.1% |
| Heparin lithium sample-8 | 0.477 | 0.443 | -7.1% |
| Heparin lithium sample-9 | 0.389 | 0.359 | -7.7% |
| Heparin lithium sample-10 | 0.362 | 0.337 | -6.8% |
| | | Mean variation | -4.6% |

| Heparin lithium-sample stability (2~8 °C) | | | |
|---|---|---|---|
| b-CTx_R_rec_132 and b-CTx_R_rec_191 | 0 h concentration-ng/mL | 24 h concentration-ng/mL | Variation |
| Heparin lithium sample-11 | 0.690 | 0.655 | -5.0% |
| Heparin lithium sample-12 | 0.476 | 0.486 | 2.0% |
| Heparin lithium sample-13 | 0.944 | 0.864 | -8.4% |
| Heparin lithium sample-14 | 0.351 | 0.380 | 8.1% |
| Heparin lithium sample-15 | 0.702 | 0.663 | -5.5% |
| Heparin lithium sample-16 | 0.175 | 0.187 | 6.7% |
| Heparin lithium sample-17 | 0.391 | 0.386 | -1.1% |
| Heparin lithium sample-18 | 0.208 | 0.221 | 6.6% |
| Heparin lithium sample-19 | 0.571 | 0.561 | -1.8% |
| Heparin lithium sample-20 | 0.413 | 0.422 | 2.2% |
| | | Mean variation | 0.4% |

| EDTA-K2-sample stability (20~25 °C) | | | |
|---|---|---|---|
| b-CTx_R_rec_132 and b-CTx_R_rec_191 | 0 h concentration-ng/mL | 24 h concentration-ng/mL | Variation |
| EDTA-K2 sample-1 | 0.683 | 0.652 | -4.6% |
| EDTA-K2 sample-2 | 0.247 | 0.269 | 8.9% |
| EDTA-K2 sample-3 | 0.406 | 0.376 | -7.6% |
| EDTA-K2 sample-4 | 0.135 | 0.136 | 1.2% |
| EDTA-K2 sample-5 | 0.653 | 0.645 | -1.2% |
| EDTA-K2 sample-6 | 0.163 | 0.163 | -0.3% |
| EDTA-K2 sample-7 | 0.421 | 0.390 | -7.4% |
| EDTA-K2 sample-8 | 0.425 | 0.409 | -3.7% |
| EDTA-K2 | 0.332 | 0.329 | -0.9% |

| EDTA-K2-sample stability (2~8 °C) | | | |
|---|---|---|---|
| b-CTx_R_rec_132 and b-CTx_R_rec_191 | 0 h concentration-ng/mL | 8 days concentration-ng/mL | Variation |
| EDTA-K2 sample-11 | 0.635 | 0.657 | 3.6% |
| EDTA-K2 sample-12 | 0.434 | 0.429 | -1.1% |
| EDTA-K2 sample-13 | 0.978 | 0.993 | 1.5% |
| EDTA-K2 sample-14 | 0.314 | 0.316 | 0.6% |
| EDTA-K2 sample-15 | 0.624 | 0.639 | 2.5% |
| EDTA-K2 sample-16 | 0.162 | 0.169 | 4.3% |
| EDTA-K2 sample-17 | 0.319 | 0.336 | 5.2% |
| EDTA-K2 sample-18 | 0.194 | 0.198 | 2.1% |
| EDTA-K2 | 0.514 | 0.559 | 8.8% |

| sample-9 | | | | sample-19 | | | |
|---|---|---|---|---|---|---|---|
| EDTA-K2 sample-10 | 0.290 | 0.301 | 3.8% | EDTA-K2 sample-20 | 0.387 | 0.393 | 1.6% |
| | | Mean variation | -1.2% | | | Mean variation | 2.9% |

8.4 The kits prepared using b-CTx_M_hyb_061 and b-CTx_R_rec_150 were used to detect different samples according to the measurement procedures of 8.2. The results are shown in Table 10.

Table 10

| Serum-sample stability (20~25 °C) | | | | Serum-sample stability (2~8 °C) | | | |
|---|---|---|---|---|---|---|---|
| b-CTx_M_hyb_061 and b-CTx_R_rec_150 | 0 h concentration-ng/mL | 12 h concentration-ng/mL | Variation | b-CTx_M_hyb_061 and b-CTx_R_rec_150 | 0 h concentration-ng/mL | 24 h concentration-ng/mL | Variation |
| Serum sample-1 | 0.658 | 0.715 | 8.7% | Serum sample-11 | 0.653 | 0.657 | 0.5% |
| Serum sample-2 | 0.217 | 0.205 | -5.9% | Serum sample-12 | 0.545 | 0.512 | -6.0% |
| Serum sample-3 | 0.406 | 0.398 | -2.0% | Serum sample-13 | 0.910 | 0.852 | -6.4% |
| Serum sample-4 | 0.132 | 0.139 | 5.5% | Serum sample-14 | 0.328 | 0.321 | -2.0% |
| Serum sample-5 | 0.734 | 0.689 | -6.2% | Serum sample-15 | 0.730 | 0.707 | -3.2% |
| Serum sample-6 | 0.193 | 0.180 | -6.9% | Serum sample-16 | 0.151 | 0.161 | 6.9% |
| Serum sample-7 | 0.523 | 0.484 | -7.5% | Serum sample-17 | 0.453 | 0.425 | -6.3% |
| Serum sample-8 | 0.434 | 0.468 | 7.7% | Serum sample-18 | 0.251 | 0.230 | -8.3% |
| Serum sample-9 | 0.361 | 0.338 | -6.4% | Serum sample-19 | 0.452 | 0.412 | -8.8% |
| Serum sample-10 | 0.362 | 0.337 | -6.9% | Serum sample-20 | 0.428 | 0.396 | -7.6% |
| | | Mean variation | -2.0% | | | Mean variation | -4.1% |

| Heparin lithium-sample stability (20~25 °C) | | | | Heparin lithium-sample stability (2~8 °C) | | | |
|---|---|---|---|---|---|---|---|
| b-CTx_M_hyb_061 and b-CTx_R_rec_150 | 0 h concentration-ng/mL | 12 h concentration-ng/mL | Variation | b-CTx_M_hyb_061 and b-CTx_R_rec_150 | 0 h concentration-ng/mL | 24 h concentration-ng/mL | Variation |
| Heparin lithium sample-1 | 0.765 | 0.728 | -4.8% | Heparin lithium sample-11 | 0.678 | 0.620 | -8.5% |
| Heparin lithium sample-2 | 0.244 | 0.234 | -4.4% | Heparin lithium sample-12 | 0.488 | 0.482 | -1.3% |
| Heparin lithium sample-3 | 0.416 | 0.391 | -6.0% | Heparin lithium sample-13 | 0.950 | 0.892 | -6.2% |
| Heparin lithium sample-4 | 0.129 | 0.128 | -0.7% | Heparin lithium sample-14 | 0.359 | 0.381 | 6.3% |
| Heparin lithium sample-5 | 0.756 | 0.689 | -8.8% | Heparin lithium sample-15 | 0.721 | 0.690 | -4.2% |
| Heparin lithium | 0.182 | 0.172 | -5.6% | Heparin lithium sample-16 | 0.166 | 0.155 | -6.9% |

| Heparin lithium sample-6 | | | | Heparin lithium sample-17 | 0.395 | 0.368 | -6.8% |
|---|---|---|---|---|---|---|---|
| Heparin lithium sample-7 | 0.555 | 0.509 | -8.4% | Heparin lithium sample-17 | 0.395 | 0.368 | -6.8% |
| Heparin lithium sample-8 | 0.473 | 0.440 | -7.0% | Heparin lithium sample-18 | 0.209 | 0.190 | -9.0% |
| Heparin lithium sample-9 | 0.345 | 0.358 | 3.6% | Heparin lithium sample-19 | 0.564 | 0.573 | 1.6% |
| Heparin lithium sample-10 | 0.408 | 0.374 | -8.4% | Heparin lithium sample-20 | 0.377 | 0.365 | -3.2% |
| | | Mean variation | -5.0% | | | Mean variation | -3.8% |

| EDTA-K2-sample stability (20~25 °C) | | | | EDTA-K2-sample stability (2~8 °C) | | | |
|---|---|---|---|---|---|---|---|
| b-CTx_M_hyb_061 and b-CTx_R_rec_150 | 0 h concentration-ng/mL | 24 h concentration-ng/mL | Variation | b-CTx_M_hyb_061 and b-CTx_R_rec_150 | 0 h concentration-ng/mL | 8 days concentration-ng/mL | Variation |
| EDTA-K2 sample-1 | 0.697 | 0.669 | -4.0% | EDTA-K2 sample-11 | 0.665 | 0.667 | 0.4% |
| EDTA-K2 sample-2 | 0.268 | 0.265 | -0.9% | EDTA-K2 sample-12 | 0.423 | 0.410 | -3.2% |
| EDTA-K2 sample-3 | 0.400 | 0.390 | -2.5% | EDTA-K2 sample-13 | 0.983 | 0.900 | -8.4% |
| EDTA-K2 sample-4 | 0.131 | 0.140 | 7.1% | EDTA-K2 sample-14 | 0.311 | 0.320 | 2.8% |
| EDTA-K2 sample-5 | 0.700 | 0.651 | -7.1% | EDTA-K2 sample-15 | 0.697 | 0.704 | 0.9% |
| EDTA-K2 sample-6 | 0.179 | 0.182 | 1.7% | EDTA-K2 sample-16 | 0.157 | 0.144 | -8.4% |
| EDTA-K2 sample-7 | 0.512 | 0.494 | -3.5% | EDTA-K2 sample-17 | 0.404 | 0.377 | -6.7% |
| EDTA-K2 sample-8 | 0.431 | 0.413 | -4.1% | EDTA-K2 sample-18 | 0.190 | 0.194 | 2.2% |
| EDTA-K2 sample-9 | 0.352 | 0.324 | -8.1% | EDTA-K2 sample-19 | 0.512 | 0.535 | 4.6% |
| EDTA-K2 sample-10 | 0.316 | 0.300 | -5.1% | EDTA-K2 sample-20 | 0.447 | 0.413 | -7.6% |
| | | Mean variation | -2.6% | | | Mean variation | -2.3% |

8.5 The kits prepared using b-CTx_R_rec_145 and b-CTx_R_rec_138 were used to detect different samples according to the measurement procedures of 8.2. The results are shown in Table 11.

Table 11

| Serum-sample stability (20~25 °C) | | | | Serum-sample stability (2~8 °C) | | | |
|---|---|---|---|---|---|---|---|
| b-CTx_R_rec_145 and b-CTx_R_rec_138 | 0 h concentration-ng/mL | 12 h concentration-ng/mL | Variation | b-CTx_R_rec_145 and b-CTx_R_rec_138 | 0 h concentration-ng/mL | 24 h concentration-ng/mL | Variation |
| Serum sample-1 | 0.684 | 0.623 | -8.9% | Serum sample-11 | 0.596 | 0.607 | 1.8% |
| Serum sample-2 | 0.238 | 0.220 | -7.4% | Serum sample-12 | 0.466 | 0.482 | 3.4% |
| Serum sample- | 0.389 | 0.394 | 1.4% | Serum sample- | 0.928 | 0.860 | -7.4% |

| 3 | | | |
|---|---|---|---|
| Serum sample-4 | 0.124 | 0.126 | 1.8% |
| Serum sample-5 | 0.643 | 0.604 | -6.1% |
| Serum sample-6 | 0.180 | 0.169 | -5.8% |
| Serum sample-7 | 0.468 | 0.472 | 0.7% |
| Serum sample-8 | 0.469 | 0.472 | 0.8% |
| Serum sample-9 | 0.348 | 0.344 | -1.1% |
| Serum sample-10 | 0.317 | 0.322 | 1.3% |
| | | Mean variation | -2.3% |

| 13 | | | |
|---|---|---|---|
| Serum sample-14 | 0.323 | 0.328 | 1.7% |
| Serum sample-15 | 0.629 | 0.653 | 3.7% |
| Serum sample-16 | 0.159 | 0.148 | -6.8% |
| Serum sample-17 | 0.349 | 0.328 | -5.9% |
| Serum sample-18 | 0.214 | 0.204 | -4.6% |
| Serum sample-19 | 0.484 | 0.461 | -4.9% |
| Serum sample-20 | 0.384 | 0.372 | -3.1% |
| | | Mean variation | -2.2% |

| Heparin lithium-sample stability (20~25 °C) | | | |
|---|---|---|---|
| b-CTx_R_rec_145 and b-CTx_R_rec_138 | 0 h concentration-ng/mL | 12 h concentration-ng/mL | Variation |
| Heparin lithium sample-1 | 0.702 | 0.675 | -3.8% |
| Heparin lithium sample-2 | 0.268 | 0.251 | -6.1% |
| Heparin lithium sample-3 | 0.417 | 0.388 | -6.9% |
| Heparin lithium sample-4 | 0.135 | 0.124 | -8.2% |
| Heparin lithium sample-5 | 0.735 | 0.663 | -9.7% |
| Heparin lithium sample-6 | 0.175 | 0.163 | -7.1% |
| Heparin lithium sample-7 | 0.491 | 0.478 | -2.8% |
| Heparin lithium sample-8 | 0.501 | 0.453 | -9.5% |
| Heparin lithium sample-9 | 0.410 | 0.377 | -8.2% |
| Heparin lithium sample-10 | 0.349 | 0.330 | -5.6% |
| | | Mean variation | -6.8% |

| Heparin lithium-sample stability (2~8 °C) | | | |
|---|---|---|---|
| b-CTx_R_rec_145 and b-CTx_R_rec_138 | 0 h concentration-ng/mL | 24 h concentration-ng/mL | Variation |
| Heparin lithium sample-11 | 0.677 | 0.647 | -4.5% |
| Heparin lithium sample-12 | 0.457 | 0.471 | 3.1% |
| Heparin lithium sample-13 | 0.953 | 0.884 | -7.2% |
| Heparin lithium sample-14 | 0.343 | 0.313 | -8.9% |
| Heparin lithium sample-15 | 0.687 | 0.651 | -5.3% |
| Heparin lithium sample-16 | 0.173 | 0.184 | 6.4% |
| Heparin lithium sample-17 | 0.374 | 0.344 | -8.1% |
| Heparin lithium sample-18 | 0.205 | 0.190 | -7.3% |
| Heparin lithium sample-19 | 0.567 | 0.554 | -2.2% |
| Heparin lithium sample-20 | 0.412 | 0.405 | -1.7% |
| | | Mean variation | -3.6% |

| EDTA-K2-sample stability (20~25 °C) | | | |
|---|---|---|---|
| b-CTx_R_rec_145 and b-CTx_R_rec_138 | 0 h concentration-ng/mL | 24 h concentration-ng/mL | Variation |
| EDTA-K2 sample-1 | 0.680 | 0.642 | -5.6% |
| EDTA-K2 sample-2 | 0.243 | 0.261 | 7.2% |

| EDTA-K2-sample stability (2~8 °C) | | | |
|---|---|---|---|
| b-CTx_R_rec_145 and b-CTx_R_rec_138 | 0 h concentration-ng/mL | 8 days concentration-ng/mL | Variation |
| EDTA-K2 sample-11 | 0.657 | 0.660 | 0.4% |
| EDTA-K2 sample-12 | 0.442 | 0.422 | -4.6% |

| | | | | | | | |
|---|---|---|---|---|---|---|---|
| EDTA-K2 sample-3 | 0.421 | 0.383 | -8.9% | EDTA-K2 sample-13 | 0.995 | 0.953 | -4.2% |
| EDTA-K2 sample-4 | 0.135 | 0.135 | 0.2% | EDTA-K2 sample-14 | 0.323 | 0.318 | -1.4% |
| EDTA-K2 sample-5 | 0.652 | 0.625 | -4.2% | EDTA-K2 sample-15 | 0.659 | 0.618 | -6.3% |
| EDTA-K2 sample-6 | 0.162 | 0.161 | -0.8% | EDTA-K2 sample-16 | 0.166 | 0.159 | -4.4% |
| EDTA-K2 sample-7 | 0.425 | 0.385 | -9.4% | EDTA-K2 sample-17 | 0.329 | 0.303 | -7.9% |
| EDTA-K2 sample-8 | 0.423 | 0.386 | -8.7% | EDTA-K2 sample-18 | 0.204 | 0.206 | 1.2% |
| EDTA-K2 sample-9 | 0.328 | 0.320 | -2.4% | EDTA-K2 sample-19 | 0.538 | 0.501 | -6.9% |
| EDTA-K2 sample-10 | 0.285 | 0.294 | 3.1% | EDTA-K2 sample-20 | 0.402 | 0.387 | -3.8% |
| | | Mean variation | -2.9% | | | Mean variation | -3.8% |

8.6 The kits prepared using b-CTx_M_hyb_063 and b-CTx_R_rec_138 were used to detect different samples according to the measurement procedures of 8.2. The results are shown in Table 12.

Table 12

| Serum-sample stability (20~25 °C) | | | | | Serum-sample stability (2~8 °C) | | | | |
|---|---|---|---|---|---|---|---|---|---|
| b-CTx_M_hyb_063 and b-CTx_R_rec_138 | 0 h concentration-ng/mL | 12 h concentration-ng/mL | Variation | | b-CTx_M_hyb_063 and b-CTx_R_rec_138 | 0 h concentration-ng/mL | 24 h concentration-ng/mL | Variation | |
| Serum sample-1 | 0.647 | 0.603 | -6.7% | | Serum sample-1 | 0.620 | 0.608 | -1.9% | |
| Serum sample-2 | 0.234 | 0.218 | -6.9% | | Serum sample-2 | 0.480 | 0.477 | -0.7% | |
| Serum sample-3 | 0.385 | 0.377 | -1.9% | | Serum sample-3 | 0.921 | 0.851 | -7.7% | |
| Serum sample-4 | 0.129 | 0.123 | -4.8% | | Serum sample-4 | 0.340 | 0.326 | -3.9% | |
| Serum sample-5 | 0.675 | 0.622 | -7.9% | | Serum sample-5 | 0.634 | 0.645 | 1.8% | |
| Serum sample-6 | 0.191 | 0.175 | -8.1% | | Serum sample-6 | 0.148 | 0.141 | -4.7% | |
| Serum sample-7 | 0.483 | 0.467 | -3.4% | | Serum sample-7 | 0.356 | 0.356 | 0.1% | |
| Serum sample-8 | 0.459 | 0.473 | 3.1% | | Serum sample-8 | 0.219 | 0.200 | -8.8% | |
| Serum sample-9 | 0.368 | 0.350 | -5.0% | | Serum sample-9 | 0.505 | 0.497 | -1.6% | |
| Serum sample-10 | 0.353 | 0.330 | -6.7% | | Serum sample-10 | 0.393 | 0.378 | -3.8% | |
| | | Mean variation | -4.8% | | | | Mean variation | -3.1% | |

| Heparin lithium-sample stability (20~25 °C) | | | | | Heparin lithium-sample stability (2~8 °C) | | | | |
|---|---|---|---|---|---|---|---|---|---|
| b-CTx_M_hyb_063 and b-CTx_R_rec_138 | 0 h concentration-ng/mL | 12 h concentration-ng/mL | Variation | | b-CTx_M_hyb_063 and b-CTx_R_rec_138 | 0 h concentration-ng/mL | 24 h concentration-ng/mL | Variation | |
| Heparin lithium sample-1 | 0.743 | 0.674 | -9.3% | | Heparin lithium sample-11 | 0.686 | 0.646 | -5.9% | |
| Heparin lithium sample-2 | 0.274 | 0.254 | -7.2% | | Heparin lithium sample-12 | 0.492 | 0.496 | 0.9% | |
| Heparin lithium sample-3 | 0.421 | 0.380 | -9.7% | | Heparin lithium sample-13 | 0.976 | 0.891 | -8.8% | |

| Heparin lithium sample-4 | 0.137 | 0.128 | -6.3% | Heparin lithium sample-14 | 0.349 | 0.328 | -6.2% |
| Heparin lithium sample-5 | 0.681 | 0.664 | -2.4% | Heparin lithium sample-15 | 0.659 | 0.649 | -1.5% |
| Heparin lithium sample-6 | 0.182 | 0.166 | -8.8% | Heparin lithium sample-16 | 0.175 | 0.162 | -7.3% |
| Heparin lithium sample-7 | 0.511 | 0.471 | -7.7% | Heparin lithium sample-17 | 0.376 | 0.387 | 3.0% |
| Heparin lithium sample-8 | 0.487 | 0.460 | -5.6% | Heparin lithium sample-18 | 0.209 | 0.197 | -5.7% |
| Heparin lithium sample-9 | 0.394 | 0.357 | -9.2% | Heparin lithium sample-19 | 0.537 | 0.522 | -2.9% |
| Heparin lithium sample-10 | 0.360 | 0.369 | 2.6% | Heparin lithium sample-20 | 0.437 | 0.406 | -6.9% |
| | | Mean variation | -6.4% | | | Mean variation | -4.1% |

| EDTA-K2-sample stability (20~25 °C) | | | | EDTA-K2-sample stability (2~8 °C) | | | |
|---|---|---|---|---|---|---|---|
| b-CTx_M_hyb_063 and b-CTx_R_rec_138 | 0 h concentration-ng/mL | 24 h concentration-ng/mL | Variation | b-CTx_M_hyb_063 and b-CTx_R_rec_138 | 0 h concentration-ng/mL | 8 days concentration-ng/mL | Variation |
| EDTA-K2 sample-1 | 0.686 | 0.633 | -7.7% | EDTA-K2 sample-11 | 0.630 | 0.613 | -2.6% |
| EDTA-K2 sample-2 | 0.251 | 0.269 | 7.0% | EDTA-K2 sample-12 | 0.427 | 0.421 | -1.3% |
| EDTA-K2 sample-3 | 0.420 | 0.383 | -8.8% | EDTA-K2 sample-13 | 0.984 | 0.997 | 1.3% |
| EDTA-K2 sample-4 | 0.130 | 0.136 | 4.4% | EDTA-K2 sample-14 | 0.305 | 0.284 | -6.7% |
| EDTA-K2 sample-5 | 0.640 | 0.641 | 0.2% | EDTA-K2 sample-15 | 0.632 | 0.614 | -2.8% |
| EDTA-K2 sample-6 | 0.160 | 0.162 | 0.8% | EDTA-K2 sample-16 | 0.166 | 0.168 | 1.0% |
| EDTA-K2 sample-7 | 0.415 | 0.386 | -7.1% | EDTA-K2 sample-17 | 0.328 | 0.318 | -3.0% |
| EDTA-K2 sample-8 | 0.424 | 0.410 | -3.4% | EDTA-K2 sample-18 | 0.192 | 0.193 | 0.2% |
| EDTA-K2 sample-9 | 0.333 | 0.329 | -1.3% | EDTA-K2 sample-19 | 0.508 | 0.465 | -8.5% |
| EDTA-K2 sample-10 | 0.289 | 0.299 | 3.4% | EDTA-K2 sample-20 | 0.392 | 0.361 | -8.1% |
| | | Mean variation | -1.3% | | | Mean variation | -3.0% |

8.7 The kits prepared using b-CTx_R_rec_134 and b-CTx_R_rec_145 were used to detect different samples according to the measurement procedures of 8.2. The results are shown in Table 13.

Table 13

| Serum-sample stability (20~25 °C) | | | | Serum-sample stability (2~8 °C) | | | |
|---|---|---|---|---|---|---|---|
| b-CTx_R_rec_134 and b-CTx_R_rec_145 | 0 h concentration-ng/mL | 12 h concentration-ng/mL | Variation | b-CTx_R_rec_134 and b-CTx_R_rec_145 | 0 h concentration-ng/mL | 24 h concentration-ng/mL | Variation |
| Serum sample-1 | 0.692 | 0.631 | -8.7% | Serum sample-1 | 0.616 | 0.631 | 2.3% |
| Serum sample-2 | 0.230 | 0.215 | -6.5% | Serum sample-2 | 0.483 | 0.493 | 2.0% |
| Serum sample-3 | 0.399 | 0.383 | -4.0% | Serum sample-3 | 0.925 | 0.845 | -8.7% |
| Serum sample-4 | 0.127 | 0.131 | 3.1% | Serum sample-4 | 0.324 | 0.328 | 1.1% |

| Serum sample-5 | 0.684 | 0.620 | -9.3% | Serum sample-5 | 0.660 | 0.668 | 1.2% |
|---|---|---|---|---|---|---|---|
| Serum sample-6 | 0.189 | 0.178 | -5.7% | Serum sample-6 | 0.146 | 0.144 | -1.7% |
| Serum sample-7 | 0.474 | 0.478 | 1.0% | Serum sample-7 | 0.350 | 0.332 | -5.2% |
| Serum sample-8 | 0.464 | 0.453 | -2.2% | Serum sample-8 | 0.213 | 0.207 | -3.0% |
| Serum sample-9 | 0.369 | 0.356 | -3.4% | Serum sample-9 | 0.504 | 0.484 | -4.0% |
| Serum sample-10 | 0.345 | 0.339 | -1.9% | Serum sample-10 | 0.388 | 0.367 | -5.5% |
| | | Mean variation | -3.8% | | | Mean variation | -2.1% |

| Heparin lithium-sample stability (20~25 °C) | | | | Heparin lithium-sample stability (2~8 °C) | | | |
|---|---|---|---|---|---|---|---|
| b-CTx_R_rec_134 and b-CTx_R_rec_145 | 0 h concentration-ng/mL | 12 h concentration-ng/mL | Variation | b-CTx_R_rec_134 and b-CTx_R_rec_145 | 0 h concentration-ng/mL | 24 h concentration-ng/mL | Variation |
| Heparin lithium sample-1 | 0.746 | 0.703 | -5.7% | Heparin lithium sample-11 | 0.689 | 0.660 | -4.2% |
| Heparin lithium sample-2 | 0.284 | 0.270 | -5.0% | Heparin lithium sample-12 | 0.479 | 0.491 | 2.4% |
| Heparin lithium sample-3 | 0.415 | 0.381 | -8.3% | Heparin lithium sample-13 | 0.942 | 0.884 | -6.2% |
| Heparin lithium sample-4 | 0.134 | 0.128 | -4.3% | Heparin lithium sample-14 | 0.343 | 0.321 | -6.4% |
| Heparin lithium sample-5 | 0.705 | 0.705 | 0.0% | Heparin lithium sample-15 | 0.706 | 0.678 | -3.9% |
| Heparin lithium sample-6 | 0.182 | 0.166 | -8.5% | Heparin lithium sample-16 | 0.171 | 0.159 | -7.1% |
| Heparin lithium sample-7 | 0.492 | 0.469 | -4.6% | Heparin lithium sample-17 | 0.384 | 0.378 | -1.7% |
| Heparin lithium sample-8 | 0.478 | 0.450 | -6.0% | Heparin lithium sample-18 | 0.212 | 0.220 | 3.8% |
| Heparin lithium sample-9 | 0.391 | 0.373 | -4.5% | Heparin lithium sample-19 | 0.571 | 0.569 | -0.4% |
| Heparin lithium sample-10 | 0.357 | 0.336 | -5.9% | Heparin lithium sample-20 | 0.420 | 0.432 | 2.7% |
| | | Mean variation | -5.3% | | | Mean variation | -2.1% |

| EDTA-K2-sample stability (20~25 °C) | | | | EDTA-K2-sample stability (2~8 °C) | | | |
|---|---|---|---|---|---|---|---|
| b-CTx_R_rec_134 and b-CTx_R_rec_145 | 0 h concentration-ng/mL | 24 h concentration-ng/mL | Variation | b-CTx_R_rec_134 and b-CTx_R_rec_145 | 0 h concentration-ng/mL | 8 days concentration-ng/mL | Variation |
| EDTA-K2 sample-1 | 0.663 | 0.659 | -0.7% | EDTA-K2 sample-11 | 0.575 | 0.550 | -4.3% |
| EDTA-K2 sample-2 | 0.247 | 0.226 | -8.7% | EDTA-K2 sample-12 | 0.439 | 0.463 | 5.6% |
| EDTA-K2 sample-3 | 0.405 | 0.373 | -7.9% | EDTA-K2 sample-13 | 0.930 | 0.881 | -5.2% |
| EDTA-K2 sample-4 | 0.128 | 0.133 | 3.4% | EDTA-K2 sample-14 | 0.313 | 0.300 | -4.0% |
| EDTA-K2 sample-5 | 0.663 | 0.658 | -0.8% | EDTA-K2 sample-15 | 0.637 | 0.635 | -0.4% |
| EDTA-K2 sample-6 | 0.164 | 0.164 | -0.1% | EDTA-K2 sample-16 | 0.156 | 0.145 | -6.9% |
| EDTA-K2 sample-7 | 0.412 | 0.382 | -7.3% | EDTA-K2 sample-17 | 0.302 | 0.281 | -6.7% |
| EDTA-K2 sample-8 | 0.420 | 0.408 | -3.0% | EDTA-K2 sample-18 | 0.191 | 0.197 | 3.4% |
| EDTA-K2 sample-9 | 0.321 | 0.299 | -7.0% | EDTA-K2 sample-19 | 0.512 | 0.476 | -6.9% |

| EDTA-K2 sample-10 | 0.298 | 0.295 | -1.0% | EDTA-K2 sample-20 | 0.361 | 0.347 | -4.0% |
|---|---|---|---|---|---|---|---|
| | | Mean variation | -3.3% | | | Mean variation | -3.0% |

8.8 The kits prepared using b-CTx_M_hyb_015 and b-CTx_R_rec_137 were used to detect different samples according to the measurement procedures of 8.2. The results are shown in Table 14.

Table 14

| Serum-sample stability (20~25 °C) | | | | Serum-sample stability (2~8 °C) | | | |
|---|---|---|---|---|---|---|---|
| b-CTx_M_hyb_015 and b-CTx_R_rec_137 | 0 h concentration-ng/mL | 12 h concentration-ng/mL | Variation | b-CTx_M_hyb_015 and b-CTx_R_rec_137 | 0 h concentration-ng/mL | 24 h concentration-ng/mL | Variation |
| Serum sample-1 | 0.692 | 0.631 | -8.8% | Serum sample-1 | 0.561 | 0.518 | -7.6% |
| Serum sample-2 | 0.222 | 0.215 | -3.0% | Serum sample-2 | 0.455 | 0.470 | 3.2% |
| Serum sample-3 | 0.370 | 0.386 | 4.2% | Serum sample-3 | 0.925 | 0.860 | -7.1% |
| Serum sample-4 | 0.126 | 0.127 | 1.1% | Serum sample-4 | 0.304 | 0.313 | 2.7% |
| Serum sample-5 | 0.640 | 0.610 | -4.8% | Serum sample-5 | 0.616 | 0.582 | -5.5% |
| Serum sample-6 | 0.173 | 0.166 | -4.0% | Serum sample-6 | 0.140 | 0.130 | -6.8% |
| Serum sample-7 | 0.474 | 0.453 | -4.2% | Serum sample-7 | 0.323 | 0.298 | -7.8% |
| Serum sample-8 | 0.438 | 0.416 | -5.0% | Serum sample-8 | 0.208 | 0.204 | -2.2% |
| Serum sample-9 | 0.350 | 0.324 | -7.6% | Serum sample-9 | 0.437 | 0.399 | -8.6% |
| Serum sample-10 | 0.326 | 0.315 | -3.2% | Serum sample-10 | 0.361 | 0.356 | -1.5% |
| | | Mean variation | -3.5% | | | Mean variation | -4.1% |

| Heparin lithium-sample stability (20~25 °C) | | | | Heparin lithium-sample stability (2~8 °C) | | | |
|---|---|---|---|---|---|---|---|
| b-CTx_M_hyb_015 and b-CTx_R_rec_137 | 0 h concentration-ng/mL | 12 h concentration-ng/mL | Variation | b-CTx_M_hyb_015 and b-CTx_R_rec_137 | 0 h concentration-ng/mL | 24 h concentration-ng/mL | Variation |
| Heparin lithium sample-1 | 0.706 | 0.641 | -9.2% | Heparin lithium sample-11 | 0.648 | 0.698 | 7.7% |
| Heparin lithium sample-2 | 0.263 | 0.249 | -5.4% | Heparin lithium sample-12 | 0.460 | 0.429 | -6.7% |
| Heparin lithium sample-3 | 0.399 | 0.379 | -5.1% | Heparin lithium sample-13 | 0.947 | 0.891 | -5.8% |
| Heparin lithium sample-4 | 0.135 | 0.123 | -9.3% | Heparin lithium sample-14 | 0.355 | 0.380 | 6.8% |
| Heparin lithium sample-5 | 0.690 | 0.650 | -5.9% | Heparin lithium sample-15 | 0.721 | 0.669 | -7.1% |
| Heparin lithium sample-6 | 0.171 | 0.156 | -9.0% | Heparin lithium sample-16 | 0.174 | 0.161 | -7.7% |
| Heparin lithium sample-7 | 0.466 | 0.481 | 3.2% | Heparin lithium sample-17 | 0.393 | 0.390 | -0.8% |
| Heparin lithium sample-8 | 0.436 | 0.412 | -5.6% | Heparin lithium sample-18 | 0.197 | 0.180 | -8.8% |

| Heparin lithium sample-9 | 0.363 | 0.340 | -6.4% | | Heparin lithium sample-19 | 0.571 | 0.550 | -3.7% |
|---|---|---|---|---|---|---|---|---|
| Heparin lithium sample-10 | 0.330 | 0.324 | -1.6% | | Heparin lithium sample-20 | 0.410 | 0.389 | -5.2% |
| | | Mean variation | -5.4% | | | | Mean variation | -3.1% |

| EDTA-K2-sample stability (20~25 °C) | | | | | EDTA-K2-sample stability (2~8 °C) | | | |
|---|---|---|---|---|---|---|---|---|
| b-CTx_M_hyb_015 and b-CTx_R_rec_137 | 0 h concentration-ng/mL | 24 h concentration-ng/mL | Variation | | b-CTx_M_hyb_015 and b-CTx_R_rec_137 | 0 h concentration-ng/mL | 8 days concentration-ng/mL | Variation |
| EDTA-K2 sample-1 | 0.692 | 0.653 | -5.7% | | EDTA-K2 sample-11 | 0.604 | 0.584 | -3.2% |
| EDTA-K2 sample-2 | 0.246 | 0.228 | -7.5% | | EDTA-K2 sample-12 | 0.428 | 0.426 | -0.4% |
| EDTA-K2 sample-3 | 0.411 | 0.385 | -6.4% | | EDTA-K2 sample-13 | 0.951 | 0.921 | -3.2% |
| EDTA-K2 sample-4 | 0.135 | 0.135 | 0.1% | | EDTA-K2 sample-14 | 0.314 | 0.295 | -6.1% |
| EDTA-K2 sample-5 | 0.644 | 0.664 | 3.1% | | EDTA-K2 sample-15 | 0.638 | 0.604 | -5.4% |
| EDTA-K2 sample-6 | 0.156 | 0.144 | -7.4% | | EDTA-K2 sample-16 | 0.161 | 0.151 | -5.9% |
| EDTA-K2 sample-7 | 0.415 | 0.395 | -4.8% | | EDTA-K2 sample-17 | 0.320 | 0.342 | 6.9% |
| EDTA-K2 sample-8 | 0.423 | 0.412 | -2.5% | | EDTA-K2 sample-18 | 0.194 | 0.196 | 1.2% |
| EDTA-K2 sample-9 | 0.332 | 0.331 | -0.4% | | EDTA-K2 sample-19 | 0.512 | 0.525 | 2.5% |
| EDTA-K2 sample-10 | 0.289 | 0.269 | -7.1% | | EDTA-K2 sample-20 | 0.383 | 0.364 | -5.0% |
| | | Mean variation | -3.9% | | | | Mean variation | -1.9% |

At present, the requirements of β-CTx immunodiagnostic kits commercially available from international manufacturers for sample stability are as follows:

1. Serum: samples should be used within 6 h if stored at 20~25 °C, and samples should be used within 8 h if stored at 2~8 °C;
2. Heparin lithium plasma: samples should be used within 4 h if stored at 20~25 °C, and samples should be used within 8 h if stored at 2~8 °C;
3. EDTA-K2 plasma: samples should be used within 24 h if stored at 20~25°C, and samples should be used within 8 days if stored at 2~8 °C.

It can be seen from Table 9-Table 14 that compared with the β-CTx immunodiagnostic kits commercially available from international manufacturers, the kits prepared by the antibodies of the present application reduce the requirements for storage time of samples, with the following details:

1. Serum: the storage time of samples can be extended to 12 h at 20~25°C, and extended to 24 h at 2~8°C;
2. Heparin lithium plasma: the storage time of samples can be extended to 12 h at 20~25°C, and extended to 24 h at 2~8°C;
3. EDTA-K2 plasma: samples of 24 h can be used if stored at 20~25°C and samples of 8 days can be used if stored at 2~8°C.

**Example 9 Assessment on consistency between serum, heparin lithium plasma and EDTA-K2 plasma samples**

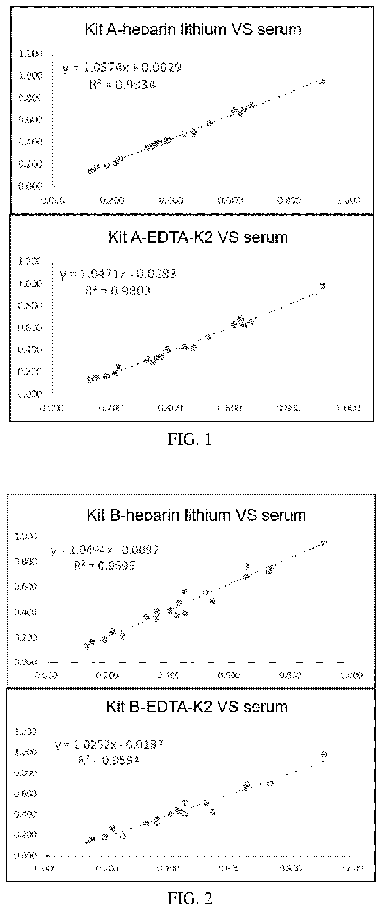

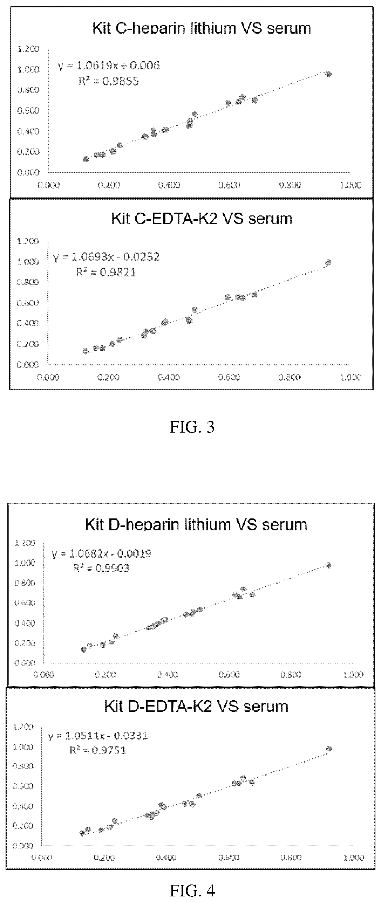

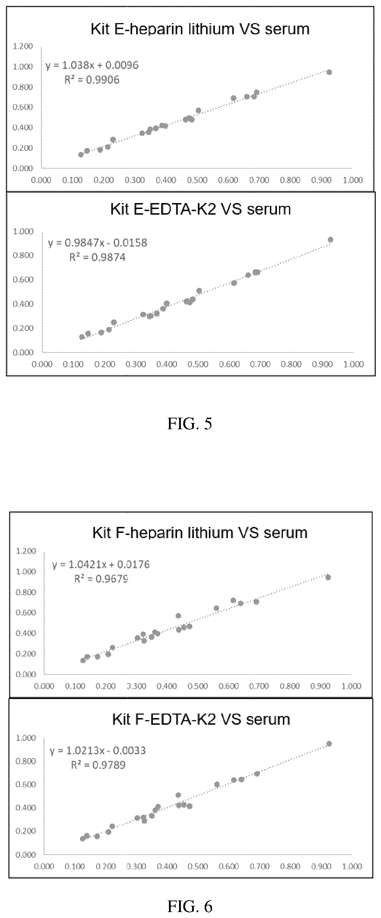

Next, the kits in Example 8 were assessed for the consistency between the results of for different sample types, and the results are shown in FIGs. 1-6.

It can be seen from FIGs. 1-6 that the kits prepared using antibodies of the present application have high consistency when testing different types of samples.

**Example 10 Assessment on specificity**

In addition to the previously verified $\alpha$-CTx, the anti-interference ability of the antibodies of the present application was further verified. In this experiment, common endogenous interfering substances including N-terminal midfragment of osteocalcin (N-MID OC), Type 1 procollagen N-terminal propeptide (P1NP) and parathyroid hormone (PTH) were tested, and serum samples containing the above interfering substances were prepared. The test procedures were performed as described in Example 8.

The cross-reactivity percentage was calculated from the measured concentration/supplemented concentration.

The test results are shown in Table 15.

Table 15

| Cross-reactivity (%) | N-MID OC | P1NP | PTH |
|---|---|---|---|
| Kit A | 0.000% | 0.001% | 0.000% |
| Kit B | 0.003% | 0.001% | 0.000% |
| Kit C | 0.012% | 0.000% | 0.000% |
| Kit D | 0.009% | 0.000% | 0.000% |
| Kit E | 0.005% | 0.001% | 0.000% |
| Kit F | 0.001% | 0.000% | 0.000% |

It can be seen from Table 15 that the kits of the present application have low cross-reactivity with common $\beta$-CTx analogues and have good specificity.

**Example 11 Assessment on detection range and linearity**

A quantitative determination method that established a linear range was used, and 11 samples with known concentration levels were selected within the expected determination range. The sample with the highest concentration close to the upper limit of the linear range was diluted to several concentrations at a certain ratio, and the sample with the lowest concentration was close to the lower limit of the linear range. The measurement results and the theoretical results were linearly fitted, and the correlation coefficient $R^2$ was calculated in the linear range.

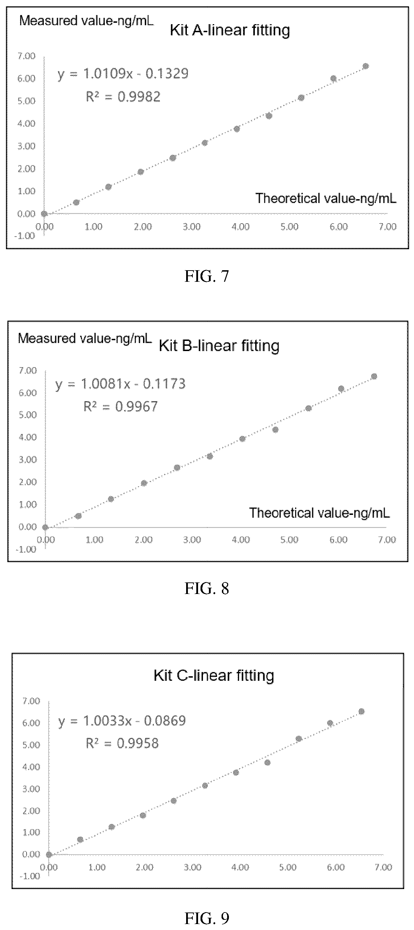

The results show that: the determination ranges of the kits of the present application all can reach 6.00 ng/mL or above, and the linear $R^2$ is greater than 0.99 between 0 ng/mL and 6.00 ng/mL, meeting the performance requirements of the kits. FIGs. 7-9 show exemplary fitting results.

**Example 12 Assessment on clinical diagnosis effectiveness**

The gold standard for the diagnosis of osteoporosis is bone mineral density. However, by the measurement and analysis of bone turnover markers, the type of osteoporosis can be further clarified, which plays a crucial role in guiding clinicians' treatment decisions.

Osteoporosis can be classified into high-turnover osteoporosis and low-turnover osteoporosis according to the bone turnover rate. Generally, for patients with high-turnover osteoporosis, anti-bone resorption therapeutic regimens are usually given clinically. For patients with low-turnover osteoporosis, basic calcium agents or therapeutic regimens that promote bone formation will be given clinically.

Patient 1:
female, 63 years old, with GRF (glomerular filtration rate) of 93.6 ml/min that is normal, blood calcium level of 2.19 mmol/L that is normal, blood phosphorus level of 1.31 mmol/L that is normal, PTH (parathyroid hormone) level of 72.29 pg/ml that is normal, VD-T (total 25-OH vitamin D) of 23.26 ng/ml that is insufficient, TP1NP (total Type 1 procollagen N-terminal propeptide) of 86.71 ng/ml that is of high turnover type, and the patient's clinical manifestation was waist and back pain for 6 months.

Based on the above information, the clinical diagnosis result was primary high-turnover osteoporosis.

According to the relevant clinical guidelines for osteoporosis, the $\beta$-CTx level exceeding 1.0 ng/ml indicates high-turnover osteoporosis. $\beta$-CTx test was performed using kit C, the test result was 1.317 ng/ml, confirming diagnosis of high-turnover osteoporosis. This indicates that the diagnostic conclusion based on the test results of the β-CTx kit of the present application is consistent with the clinical diagnosis result.

Patient 2:

female, 62 years old, with GRF (glomerular filtration rate) of 100.53 ml/min that is normal, blood calcium level of 2.28 mmol/L that is normal, blood phosphorus level of 1.26 mmol/L that is normal, PTH (parathyroid hormone) level of 40.97 pg/ml that is normal, VD-T (total 25-OH vitamin D) of 21.25 ng/ml that is insufficient, TP1NP (total Type 1 procollagen N-terminal propeptide) of 123.35 ng/ml that is of high turnover type, and the patient's clinical manifestation was waist and back pain for 8 months.

Based on the above information, the clinical diagnosis result was primary high-turnover osteoporosis.

β-CTx test was performed using kit C, and the test result was 1.256 ng/ml, confirming diagnosis of high-turnover osteoporosis according to the relevant clinical guidelines. This indicates that the diagnostic conclusion based on the test results of the β-CTx kit of the present application is consistent with the clinical diagnosis result.

Patient 3:

male, 78 years old, with GRF (glomerular filtration rate) of 101.98 ml/min that is normal, blood calcium level of 2.17 mmol/L that is normal, blood phosphorus level of 1.01 mmol/L that is normal, PTH (parathyroid hormone) level of 67.85 pg/ml that is normal, VD-T (total 25-OH vitamin D) of 34.66 ng/ml that is normal, TP1NP (total Type 1 procollagen N-terminal propeptide) of 16.48 ng/ml that is of low turnover type, and the patient's clinical manifestation was waist and back pain for 9 months.

Based on the above information, the clinical diagnosis result was primary low-turnover osteoporosis.

β-CTx test was performed using kit C, and the test result was 0.043 ng/ml, confirming diagnosis of low-turnover osteoporosis according to the relevant clinical guidelines. This indicates that the diagnostic conclusion based on the test results of the β-CTx kit of the present application is consistent with the clinical diagnosis result.

This example shows that the diagnosis results of osteoporosis based on the test results of the β-CTx kits of the present application are consistent with the clinical diagnosis results obtained by comprehensive evaluation of multiple biochemical indicators and the clinical manifestations of patients.

## Claims

1. An antibody or an antigen-binding fragment thereof capable of binding to β-CTx, comprising:

a heavy chain variable region (VH) and a light chain variable region (VL),
wherein the heavy chain variable region comprises HCDR1 including an amino acid sequence as set forth in any one of SEQ ID NO: 1, 7, 13, 19, 25, 31, 37, 43, 49 or 55 or a variant thereof; HCDR2 including an amino acid sequence as set forth in any one of SEQ ID NO: 2, 8, 14, 20, 26, 32, 38, 44, 50 or 56 or a variant thereof; and HCDR3 including an amino acid sequence as set forth in any one of SEQ ID NO: 3, 9, 15, 21, 27, 33, 39, 45, 51 or 57 or a variant thereof;
wherein, the light chain variable region comprises LCDR1 including an amino acid sequence as set forth in any one of SEQ ID NO: 4, 10, 16, 22, 28, 34, 40, 46, 52 or 58 or a variant thereof; LCDR2 including an amino acid sequence as set forth in any one of SEQ ID NO: 5, 11, 17, 23, 29, 35, 41, 47, 53 or 59 or a variant thereof; and LCDR3 including an amino acid sequence as set forth in any one of SEQ ID NO: 6, 12, 18, 24, 30, 36, 42, 48, 54 or 60 or a variant thereof; and
wherein each variant comprises one or more amino acid mutations compared to the amino acid sequence from which it is derived, and each amino acid mutation is selected from amino acid substitution, deletion or addition; preferably, the substitution is a conservative substitution; preferably, each variant has at least 80%, at least 90%, or at least 95% identity to the amino acid sequence from which it is derived;
preferably, the antibody or the antigen-binding fragment thereof recognize an antigen-binding epitope selected from one or more of the group consisting of: AH(betaD)G, AH(betaD)GG, AH(betaD)GGR and AH(betaD)GGR-COOH.

2. The antibody or the antigen-binding fragment thereof according to claim 1, wherein:

HCDR1, HCDR2, HCDR3, LCDR1, LCDR2 and LCDR3 include sequences that have at least 80%, at least 90%, or at least 95% identity to SEQ ID NOs: 1-6, respectively;
HCDR1, HCDR2, HCDR3, LCDR1, LCDR2 and LCDR3 include sequences that have at least 80%, at least 90%, or at least 95% identity to SEQ ID NOs: 7-12, respectively;
HCDR1, HCDR2, HCDR3, LCDR1, LCDR2 and LCDR3 include sequences that have at least 80%, at least 90%, or at least 95% identity to SEQ ID NOs: 13-18, respectively;

HCDR1, HCDR2, HCDR3, LCDR1, LCDR2 and LCDR3 include sequences that have at least 80%, at least 90%, or at least 95% identity to SEQ ID NOs: 19-24, respectively;
HCDR1, HCDR2, HCDR3, LCDR1, LCDR2 and LCDR3 include sequences that have at least 80%, at least 90%, or at least 95% identity to SEQ ID NOs: 25-30, respectively;
HCDR1, HCDR2, HCDR3, LCDR1, LCDR2 and LCDR3 include sequences that have at least 80%, at least 90%, or at least 95% identity to SEQ ID NOs: 31-36, respectively;
HCDR1, HCDR2, HCDR3, LCDR1, LCDR2 and LCDR3 include sequences that have at least 80%, at least 90%, or at least 95% identity to SEQ ID NOs: 37-42, respectively;
HCDR1, HCDR2, HCDR3, LCDR1, LCDR2 and LCDR3 include sequences that have at least 80%, at least 90%, or at least 95% identity to SEQ ID NOs: 43-48, respectively;
HCDR1, HCDR2, HCDR3, LCDR1, LCDR2 and LCDR3 include sequences that have at least 80%, at least 90%, or at least 95% identity to SEQ ID NOs: 49-54, respectively; or
HCDR1, HCDR2, HCDR3, LCDR1, LCDR2 and LCDR3 include sequences that have at least 80%, at least 90%, or at least 95% identity to SEQ ID NOs: 55-60, respectively.

3. The antibody or the antigen-binding fragment thereof according to claim 1, wherein:

HCDR1, HCDR2, HCDR3, LCDR1, LCDR2 and LCDR3 include sequences as set forth in SEQ ID NOs: 1-6, respectively;
HCDR1, HCDR2, HCDR3, LCDR1, LCDR2 and LCDR3 include sequences as set forth in SEQ ID NOs: 7-12, respectively;
HCDR1, HCDR2, HCDR3, LCDR1, LCDR2 and LCDR3 include sequences as set forth in SEQ ID NOs: 13-18, respectively;
HCDR1, HCDR2, HCDR3, LCDR1, LCDR2 and LCDR3 include sequences as set forth in SEQ ID NOs: 19-24, respectively;
HCDR1, HCDR2, HCDR3, LCDR1, LCDR2 and LCDR3 include sequences as set forth in SEQ ID NOs: 25-30, respectively;
HCDR1, HCDR2, HCDR3, LCDR1, LCDR2 and LCDR3 include sequences as set forth in SEQ ID NOs: 31-36, respectively;
HCDR1, HCDR2, HCDR3, LCDR1, LCDR2 and LCDR3 include sequences as set forth in SEQ ID NOs: 37-42, respectively;
HCDR1, HCDR2, HCDR3, LCDR1, LCDR2 and LCDR3 include sequences as set forth in SEQ ID NOs: 43-48, respectively;
HCDR1, HCDR2, HCDR3, LCDR1, LCDR2 and LCDR3 include sequences as set forth in SEQ ID NOs: 49-54, respectively; or
HCDR1, HCDR2, HCDR3, LCDR1, LCDR2 and LCDR3 include sequences as set forth in SEQ ID NOs: 55-60, respectively.

4. An antibody or an antigen-binding fragment thereof, wherein the antibody is capable of being produced by hybridoma H-215 deposited with VKPM, or being produced by hybridoma H-216 deposited with VKPM, or being produced by hybridoma H-217 deposited with VKPM.

5. An antibody or an antigen-binding fragment thereof, wherein the antibody:

is produced based on a plasmid deposited with VKPM under a deposit number of B-14772 and a plasmid deposited with VKPM under a deposit number of B-14773;
is produced based on a plasmid deposited with VKPM under a deposit number of B-14774 and a plasmid deposited with VKPM under a deposit number of B-14775;
is produced based on a plasmid deposited with VKPM under a deposit number of B-14776 and a plasmid deposited with VKPM under a deposit number of B-14777;
is produced based on a plasmid deposited with VKPM under a deposit number of B-14778 and a plasmid deposited with VKPM under a deposit number of B-14779;
is produced based on a plasmid deposited with VKPM under a deposit number of B-14780 and a plasmid deposited with VKPM under a deposit number of B-14781;
is produced based on a plasmid deposited with VKPM under a deposit number of B-14782 and a plasmid deposited with VKPM under a deposit number of B-14783; or
is produced based on a plasmid deposited with VKPM under a deposit number of B-14784 and a plasmid deposited with VKPM under a deposit number of B-14785.

**EP 4 674 867 A2**

6. An isolated nucleic acid molecule encoding the antibody or the antigen-binding fragment thereof according to any one of claims 1-5.

7. An expression vector comprising the isolated nucleic acid molecule according to claim 6.

8. A host cell comprising the isolated nucleic acid molecule according to claim 6 or the expression vector according to claim 7.

9. A kit, wherein the kit is for detecting β-CTx in a sample and the kit comprises a first antibody or an antigen-binding fragment thereof and a second antibody or an antigen-binding fragment thereof, wherein the first antibody or the antigen-binding fragment thereof or the second antibody or the antigen-binding fragment thereof is selected from the antibody or the antigen-binding fragment thereof of any one of claims 1 to 3;

preferably, the first antibody is a capture antibody, and the second antibody is a detection antibody;
preferably, the sample is serum or plasma, such as heparin plasma or EDTA plasma, preferably serum or plasma collected after 8 h, 12 h, 24 h, 2 days, 4 days, or 8 days;
preferably, a cross-reactivity percentage of the kit with α-Ctx is not higher than 0.20%, such as not higher than 0.10%, 0.05%, or 0.02%.

10. The kit according to claim 9, wherein the first antibody is different from the second antibody, and the first antibody and the second antibody each independently comprises:

HCDR1, HCDR2, HCDR3, LCDR1, LCDR2 and LCDR3 including sequences that have at least 80%, at least 90%, at least 95%, or 100% identity to SEQ ID NOs: 1-6, respectively;
HCDR1, HCDR2, HCDR3, LCDR1, LCDR2 and LCDR3 including sequences that have at least 80%, at least 90%, at least 95%, or 100% identity to SEQ ID NOs: 7-12, respectively;
HCDR1, HCDR2, HCDR3, LCDR1, LCDR2 and LCDR3 including sequences that have at least 80%, at least 90%, at least 95%, or 100% identity to SEQ ID NOs: 13-18, respectively;
HCDR1, HCDR2, HCDR3, LCDR1, LCDR2 and LCDR3 including sequences that have at least 80%, at least 90%, at least 95%, or 100% identity to SEQ ID NOs: 19-24, respectively;
HCDR1, HCDR2, HCDR3, LCDR1, LCDR2 and LCDR3 including sequences that have at least 80%, at least 90%, at least 95%, or 100% identity to SEQ ID NOs: 25-30, respectively;
HCDR1, HCDR2, HCDR3, LCDR1, LCDR2 and LCDR3 including sequences that have at least 80%, at least 90%, at least 95%, or 100% identity to SEQ ID NOs: 31-36, respectively;
HCDR1, HCDR2, HCDR3, LCDR1, LCDR2 and LCDR3 including sequences that have at least 80%, at least 90%, at least 95%, or 100% identity to SEQ ID NOs: 37-42, respectively;
HCDR1, HCDR2, HCDR3, LCDR1, LCDR2 and LCDR3 including sequences that have at least 80%, at least 90%, at least 95%, or 100% identity to SEQ ID NOs: 43-48, respectively;
HCDR1, HCDR2, HCDR3, LCDR1, LCDR2 and LCDR3 including sequences that have at least 80%, at least 90%, at least 95%, or 100% identity to SEQ ID NOs: 49-54, respectively; or
HCDR1, HCDR2, HCDR3, LCDR1, LCDR2 and LCDR3 including sequences that have at least 80%, at least 90%, at least 95%, or 100% identity to SEQ ID NOs: 55-60, respectively.

11. The kit according to claim 10, wherein

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 1-6, respectively;
the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 1-6, respectively;
the first antibody comprises HCDR1, HCDR2, HCDR3, LCDR1, LCDR2 and LCDR3 including sequences as set forth in SEQ ID NOs: 1-6, respectively; and the second antibody comprises HCDR1, HCDR2, HCDR3, LCDR1, LCDR2 and LCDR3 including sequences as set forth in SEQ ID NOs: 7-12, respectively;
the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 25-30, respectively;
the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 37-42, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 25-30, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 25-30, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 31-36, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 31-36, respectively; and the second antibody comprises HCDR1, HCDR2, HCDR3, LCDR1, LCDR2 and LCDR3 including sequences as set forth in SEQ ID NOs: 25-30, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 49-54, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 19-24, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 25-30, respectively; or

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 25-30, respectively.

**12.** The kit according to claim 11, wherein

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 1-6, respectively; or

the first antibody comprises HCDR1, HCDR2, HCDR3, LCDR1, LCDR2 and LCDR3 including sequences as set forth in SEQ ID NOs: 1-6, respectively; and the second antibody comprises HCDR1, HCDR2, HCDR3, LCDR1, LCDR2 and LCDR3 including sequences as set forth in SEQ ID NOs: 7-12, respectively.

13. The kit according to claim 9, wherein

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 37-42, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 31-36, respectively;

the first antibody comprises HCDR1, HCDR2, HCDR3, LCDR1, LCDR2 and LCDR3 including sequences as set forth in SEQ ID NOs: 1-6, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 1-6, respectively; or

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 25-30, respectively.

14. A method for diagnosing osteoporosis in a subject, comprising:

using a first antibody or an antigen-binding fragment thereof and a second antibody or an antigen-binding fragment thereof to detect a sample from the subject;
determining the amount of $\beta$-CTx in the sample; and
determining whether the subject has osteoporosis based on the determined amount of $\beta$-CTx,
wherein one or both of the first antibody or the antigen-binding fragment thereof and the second antibody or the antigen-binding fragment thereof is selected from the antibody or the antigen-binding fragment thereof of any one of claims 1 to 3;
preferably, the first antibody and the second antibody are as defined in claim 10.

15. The method according to claim 14, wherein the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 1-6, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 25-30, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 37-42, respectively;

the first antibody comprises HCDR1, HCDR2, HCDR3, LCDR1, LCDR2 and LCDR3 including sequences as set forth in SEQ ID NOs: 43-48, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 13-18, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 19-24, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 25-30, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 25-30, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 7-12, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 1-6, respectively;

the first antibody comprises HCDR1, HCDR2, HCDR3, LCDR1, LCDR2 and LCDR3 including sequences as set forth in SEQ ID NOs: 31-36, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 31-36, respectively; and the second antibody comprises HCDR1, HCDR2, HCDR3, LCDR1, LCDR2 and LCDR3 including sequences as set forth in SEQ ID NOs: 25-30, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 13-18, respectively;

the first antibody comprises HCDR1, HCDR2, HCDR3, LCDR1, LCDR2 and LCDR3 including sequences as set forth in SEQ ID NOs: 37-42, respectively; and the second antibody comprises HCDR1, HCDR2, HCDR3, LCDR1, LCDR2 and LCDR3 including sequences as set forth in SEQ ID NOs: 49-54, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 19-24, respectively;

the first antibody comprises HCDR1, HCDR2, HCDR3, LCDR1, LCDR2 and LCDR3 including sequences as set forth in SEQ ID NOs: 49-54, respectively; and the second antibody comprises HCDR1, HCDR2, HCDR3, LCDR1, LCDR2 and LCDR3 including sequences as set forth in SEQ ID NOs: 25-30, respectively; or

the first antibody comprises HCDR1, HCDR2, HCDR3, LCDR1, LCDR2 and LCDR3 including sequences as set forth in SEQ ID NOs: 55-60, respectively; and the second antibody comprises HCDR1, HCDR2, HCDR3, LCDR1, LCDR2 and LCDR3 including sequences as set forth in SEQ ID NOs: 25-30, respectively.

FIG. 1

FIG. 2

FIG. 3

FIG. 4

FIG. 5

FIG. 6

FIG. 7

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202410890870

**Non-patent literature cited in the description**

- **CHUBB et al.** *Clinical Biochemistry*, 2012, vol. 45 (12), 928-935
- **CLOOS et al.** *Biochemistry Journal*, 2000, vol. 345, 473-480
- **KABAT et al.** Sequences of Proteins of Immunological Interest. National Institutes of Health, 1991
- **CHOTHIA et al.** *Nature*, 1989, vol. 342, 877-883
- **MACCALLIM**. *J. Mol. Biol.*, 1996, vol. 262, 732-745
- **MAKABE et al.** *Journal of Biological Chemistry*, 2008, vol. 283, 1156-1166
- **CREIGHTON**. *Proteins*, 1984
- Research Centre "Kurchatov Institute" State Research Institute "Genetika" 1-st Dorozhniy pr., 1 Moscow 117545, Russian Federation. *Russian National Collection of Industrial Microorganisms (VKPM)*, 28 March 2024
- *Russian National Collection of Industrial Microorganisms (VKPM)*, 28 March 2024
- *VKPM*, 02 April 2024
- *VKPM*, 28 March 2024
- *t VKPM*, 02 April 2024
- Evaluation of Detection Capability for Clinical Laboratory Measurement Procedures. Approved Guideline. Clinical and Laboratory Standards Institute (CLSI), vol. 24